# EUROPÄISCHE PATENTSCHRIFT

(11) **EP 1 599 460 B1**
(45) Veröffentlichungstag und Bekanntmachung des Hinweises auf die Patenterteilung: **07.11.2007**
(21) Anmeldenummer: 04708349.8
(22) Anmeldetag: 05.02.2004
(51) Int. Cl.: C07D 327/06, A01N 43/84

(54) **OXATHIINCARBOXAMIDE**
OXATHIIN CARBOXAMIDE
OXATHIINE-CARBOXAMIDES

(30) Priorität: 14.02.2003 DE 10306244; 13.05.2003 DE 10321270
(43) Veröffentlichungstag der Anmeldung: 30.11.2005
(73) Patentinhaber: Bayer CropScience Aktiengesellschaft, 40789 Monheim (DE)
(72) Erfinder: DUNKEL, Ralf, 69001 Lyon (FR); ELBE, Hans-Ludwig, 42329 Wuppertal (DE); RIECK, Heiko, 69110 Ste Foy Les Lyon (FR); GREUL, Nico, Jörg, 42799 Leichlingen (DE); WACHENDORFF-NEUMANN, Ulrike, 56566 Neuwied (DE); DAHMEN, Peter, 41470 Neuss (DE); KUCK, Karl-Heinz, 40764 Langenfeld (DE); HARTMANN, Benoit, 40223 Langenfeld (DE)
(86) Internationale Anmeldenummer: PCT/EP2004/001053
(87) Internationale Veröffentlichungsnummer: WO 2004/072023

(56) Entgegenhaltungen:
- EP-A- 0 545 099
- US-A- 3 657 449
- HAHN, HOH-GYU ET AL: "Synthesis of trifluoromethylated dihydro-1,4-oxathiincarboxanilides and their fungicidal activity" HAN'GUK NONGHWA HAKHOECHI , 44(3), 191-196 CODEN: JKACA7; ISSN: 0368-2897, 2001, XP009038028
- HAHN, HOH-GYU ET AL: "Synthesis of trifluoromethylated dihydro-1,4-oxathiin-3-carboxanilides through polymer-bound activated ester" HETEROCYCLES , 48(11), 2253-2261 CODEN: HTCYAM; ISSN: 0385-5414, 1998, XP009038022

## Beschreibung

Die vorliegende Erfindung betrifft neue Oxathiincarboxamide, mehrere Verfahren zu deren Herstellung und deren Verwendung zur Bekämpfung von unerwünschten Mikroorganismen.

Es ist bereits bekannt, dass zahlreiche Carboxamide fungizide Eigenschaften besitzen (vgl. z.B. EP-A 0 591 699, EP-A 0 545 099, DE-OS 16 17 921, JP-A 2001-302605, JP-A 10-251240, JP-A 8-176112, JP-A 53-72823 und US 3,657,449).

So sind bereits einige 6-Methyl-2,3-dihydro-1,4-oxathiin-5-carboxamide bekannt geworden. Beispielhaft seien genannt *N*-(4'-Fluor-1,1'-biphenyl-2-yl)-6-methyl-2,3-dihydro-1,4-oxathiin-5-carboxamid und (6-Methyl(2,3-dihydro-1,4-oxathiin-5-yl))-*N*-[2-(2-methylpropyl)phenyl]-carboxamid aus EP-A 0 545 099, (6-Methyl(2,3-dihydro-1,4-oxathiin-5-yl))-*N*-(2,4,6-trimethylphenyl)carboxamid aus DE-OS 16 17 921, *N*-[2-(1,3-Dimethylbutyl)phenyl](6-methyl(2,3-dihydro-1,4-oxathiin-5-yl))carboxamid aus JP-A 10-251240 und (6-Methyl(2,3-dihydro-1,4-oxathiin-5-yl))-*N*-(2-methylphenyl)carboxamid aus US 3,657,449. Die Wirksamkeit dieser Stoffe ist gut, lässt aber in manchen Fällen, z.B. bei niedrigen Aufwandmengen zu wünschen übrig.

Es wurden nun neue Oxathiincarboxamide der Formel (I) 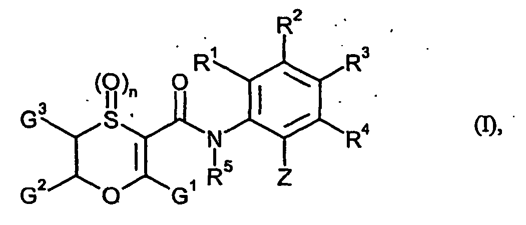 gefunden, in welcher
- G¹: für Halogen, Trifluormethyl, Difluormethyl oder Cyclopropyl steht,
- G² und G³: unabhängig voneinander für Wasserstoff oder Methyl stehen,
- n: für 0, 1 oder 2 steht,
- R¹, R², R³ und R⁴: unabhängig voneinander für Wasserstoff, Fluor, Chlor, Methyl, iso-Propyl oder Methylthio stehen,
- R⁵: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenakylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁶, -CONR⁷R⁸ oder -CH₂NR⁹R¹⁰ steht,
- R⁶: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-akoxy-C₁-C₄-alkyl, C₃-C₈-Halogencycloalkyl mit jeweils 1 bis 9 Fluor-, Chlor- und/oder Bromatomen; -COR¹¹ steht,
- R⁷ und R⁸: unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
- R⁷ und R⁸: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
- R⁹ und R¹⁰: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
- R⁹ und R¹⁰: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
- R¹¹: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R¹²: für Wasserstoff oder C₁-C₆-Alkyl steht,
- Z: für Z¹, Z², Z³ oder Z⁴ steht, worin
Z¹ für einfach bis fünffach, gleich oder verschieden substituiertes Phenyl steht,
- Z²: für gegebenenfalls einfach oder mehrfach, gleich oder verschieden substituiertes Cycloalkyl oder Bicycloalkyl steht,
- Z³: für unsubstituiertes C₄-C₂₀-Alkyl oder für einfach oder mehrfach, gleich oder verschieden durch Halogen und/oder C₃-C₆-Cycloalkyl substituiertes C₁-C₂₀-Alkyl steht, wobei der Cycloalkylteil seinerseits gegebenenfalls einfach oder mehrfach, gleich oder verschieden durch Halogen und/oder C₁-C₄-Alkyl substituiert sein kann,
- Z⁴: für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann,
oder
- R¹, R² und R³: unabhängig voneinander für Wasserstoff oder Fluor stehen und
- Z und: R⁴ gemeinsam mit den Kohlenstoffatomen, an die sie gebunden sind, einen gegebenenfalls substituierten 5- oder 6-gliedrigen carbocyclischen oder heterocyclischen Ring bilden.

Weiterhin wurde gefunden, dass man Oxathiincarboxamide der Formel (I) erhält, indem man
a) Oxathiincarbonsäurederivate der Formel (II) 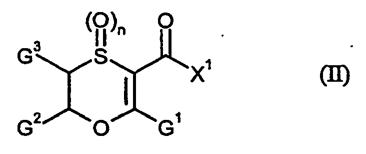 in welcher
   - G¹, G², G³ und n: die oben angegebenen Bedeutungen haben und
   - X¹: für Halogen oder Hydroxy steht,
   mit Anilin-Derivaten der Formel (III) 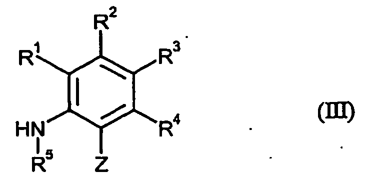 in welcher
   - R¹, R², R³, R⁴, R⁵ und Z: die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder
b) Halogenoxathiincarboxamide der Formel (IV) 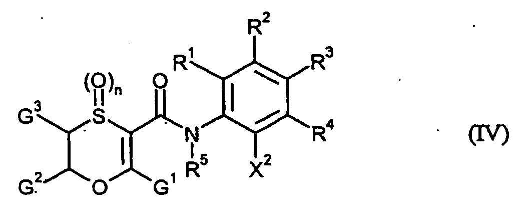 in welcher
   - G¹, G², G³, n, R¹, R², R³, R⁴ und R⁵: die oben angegebenen Bedeutungen haben,
   - X²: für Brom oder Iod steht,
   mit Boronsäure-Derivaten der Formel (V) 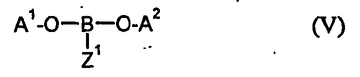 in welcher
   - Z¹: die oben angegebenen Bedeutungen hat und
   - A¹ und A²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
c) Oxathiincarboxamid-Boronsäure-Derivate der Formel (VI) 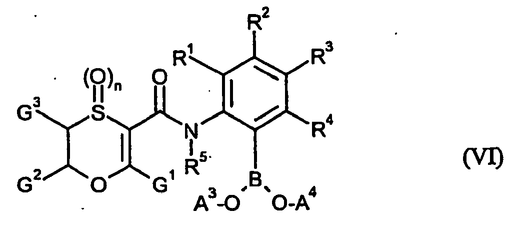 in welcher
   - G¹, G², G³, n, R¹, R², R³, R⁴ und R⁵: die oben angegebenen Bedeutungen haben,
   - A³ und A⁴: jeweils für Wasserstoff oder zusammen für Tetramethylethylen stehen,
   mit Phenyl-Derivaten der Formel (VII)

   X³-Z¹ (VII)

   in welcher
   - Z¹: die oben angegebenen Bedeutungen hat und
   - X³: für Chlor, Brom, Iod oder Trifluormethylsulfonat steht,
   in Gegenwart eines Katalysators, gegebenenfalls in Gegenwart eines Säurebindemittels und gegebenenfalls in Gegenwart eines Verdünnungsmittels umsetzt, oder
d) Halogenoxathiincarboxamide der Formel (IV) 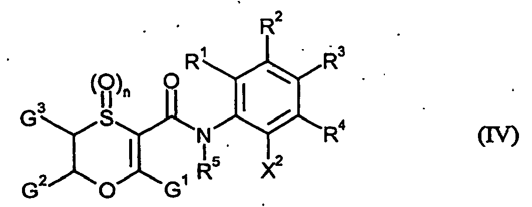 in welcher
   - G¹, G², G³, n, R¹, R², R³, R⁴ und R⁵: die oben angegebenen Bedeutungen haben,
   - X²: für Brom oder Iod steht,
   mit Phenyl-Derivaten der Formel (VII)

   X³-Z¹ (VII)

   in welcher
   - Z¹: die oben angegebenen Bedeutungen hat und
   - X³: für Chlor, Brom, Iod oder Trifluormethylsulfonat steht,
   in Gegenwart eines Palladium- oder Nickel-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt, oder
e) Oxathiincarboxamide der Formel (Ia) 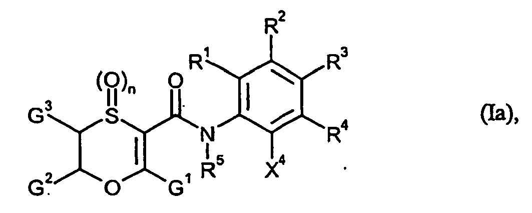 in welcher
   - G¹, G², G³, n, R¹, R², R³, R⁴ und R⁵: die oben angegebenen Bedeutungen haben,
   - X⁴: für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart eines Katalysators hydriert, oder
f) Hydroxyalkyloxathiincarboxamide der Formel (VIII) 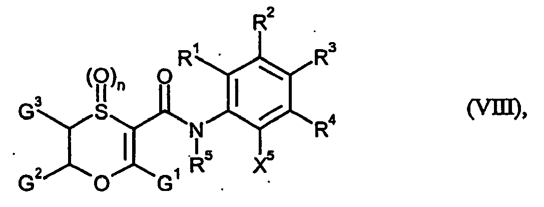 in welcher
   - G¹, G², G³, n, R¹, R², R³, R⁴ und R⁵: die oben angegebenen Bedeutungen haben,
   - X⁵: für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart einer Säure dehydratisiert, oder
g) Halogenoxathiincarboxamide der Formel (IV) 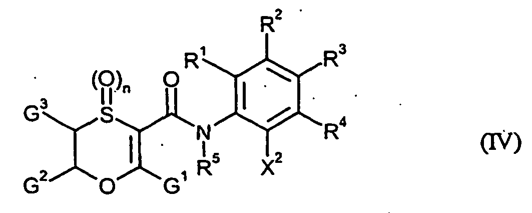 in welcher
   - G¹, G², G³, n, R¹, R², R³, R⁴ und R⁵: die oben angegebenen Bedeutungen haben,
   - X²: für Brom oder Iod steht,
   mit einem Alkin der Formel (IX)

   HC≡-A⁵ (IX),

   in welcher
   - A⁵: für jeweils gegebenenfalls einfach oder mehrfach, gleich oder verschieden durch Halogen und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
   oder einem Alken der Formel (X) 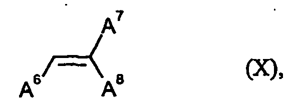 in welcher
   - A⁶, A⁷ und A⁸: unabhängig voneinander jeweils für Wasserstoff oder gegebenenfalls einfach oder mehrfach, gleich oder verschieden durch Halogen und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 20 nicht übersteigt,
   gegebenenfalls in Gegenwart eines Verdünnungsmittels, gegebenenfalls in Gegenwart eines Säurebindemittels und in Gegenwart eines oder mehrerer Katalysatoren umsetzt, oder
h) Ketone der Formel (XI) 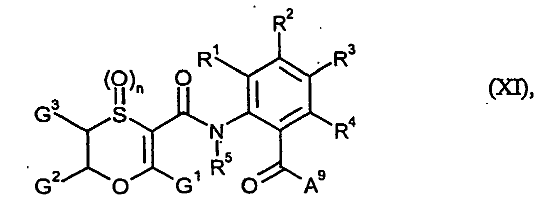 in welcher
   - G¹, G², G³, n, R¹, R², R³, R⁴ und R⁵: die oben angegebenen Bedeutungen haben,
   - A⁹: für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   mit einer Phosphorverbindung der allgemeinen Formel (XII)

   A¹⁰-Px (XII),

   in welcher
   - A¹⁰: für gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   - Px: für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃ steht,
   gegebenenfalls in Gegenwart eines Verdünnungsmittels umsetzt, oder
i) Oxathiincarboxamide der Formel (Ib) 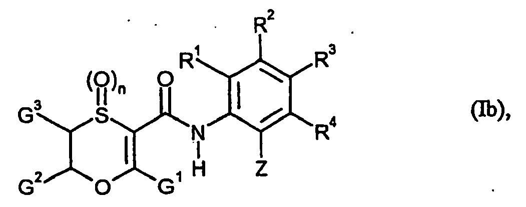 in welcher
   - G¹, G², G³, n, R¹, R², R³, R⁴ und Z: die oben angegebenen Bedeutungen haben,
   mit Halogeniden der Formel (XIII)

   R⁵⁻¹-X⁶ (XIII)

   in welcher
   - R⁵⁻¹: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁶, -CONR⁷R⁸ oder -CH₂NR⁹R¹⁰ steht,
   - R⁶, R⁷, R⁸, R⁹ und R¹⁰: die oben angegebenen Bedeutungen haben,
   - X⁶: für Chlor, Brom oder Iod steht,
   in Gegenwart einer Base und in Gegenwart eines Verdünnungsmittels umsetzt.

Schließlich wurde gefunden, dass die neuen Oxathiincarboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Oxathiincarboxamide der Formel (I) eine wesentlich bessere fungizide Wirksamkeit als die konstitutionell ähnlichsten, vorbekannten Wirkstoffe gleicher Wirkungsrichtung.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Oxathiincarboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- G¹: steht bevorzugt für Fluor, Chlor, Brom, Iod, Trifluormethyl, Difluormethyl oder Cyclopropyl.
- G¹: steht besonders bevorzugt für Chlor, Brom, Iod, Trifluormethyl, Difluormethyl oder Cyclopropyl.
- G¹: steht ganz besonders bevorzugt für Trifluormethyl.
- G¹: steht auch ganz besonders bevorzugt für Difluormethyl.
- G¹: steht auch ganz besonders bevorzugt für Cyclopropyl.
- G²: steht bevorzugt für Wasserstoff.
- G²: steht auch bevorzugt für Methyl.
- G³: steht bevorzugt für Wasserstoff.
- G³: steht auch bevorzugt für Methyl.
- G² und G³: stehen besonders bevorzugt gleichzeitig für Wasserstoff.

- n: steht bevorzugt 0 oder 2.
- n: steht besonders bevorzugt für 0.
- n: steht auch besonders bevorzugt für 2.

- R¹: steht bevorzugt für Wasserstoff, Fluor, Chlor oder Methyl.
- R¹: steht besonders bevorzugt für Wasserstoff, Fluor oder Chlor.
- R¹: steht ganz besonders bevorzugt für Wasserstoff.
- R¹: steht auch ganz besonders bevorzugt für Fluor.
- R²: steht bevorzugt für Wasserstoff, Fluor, Chlor, iso-Propyl oder Methylthio.
- R²: steht besonders bevorzugt für Wasserstoff, Fluor, iso-Propyl oder Methylthio.
- R²: steht ganz besonders bevorzugt für Wasserstoff.
- R³: steht bevorzugt für Wasserstoff, Fluor, Chlor oder Methyl.
- R³: steht besonders bevorzugt für Wasserstoff oder Fluor.
- R³: steht ganz besonders bevorzugt für Wasserstoff.
- R³: steht auch ganz besonders bevorzugt für Fluor.
- R⁴: steht bevorzugt für Wasserstoff, Fluor, Chlor oder Methyl.
- R⁴: steht besonders bevorzugt für Wasserstoff oder Methyl.
- R⁴: steht ganz besonders bevorzugt für Wasserstoff.
- R⁴: steht auch ganz besonders bevorzugt für Methyl.
- R¹, R², R³ und R⁴: stehen ganz besonders bevorzugt gleichzeitig für Wasserstoff.
- R⁵: steht bevorzugt für Wasserstoff; C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁶, -CONR⁷R⁸ oder -CH₂NR⁹R¹⁰.
- R⁵: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl, -CH₂-CHO, -CH₂CH₂-CHO, -CH₂-CO-CH₃, -CH₂-CO-CH₂CH₃, -CH₂-CO-CH(CH₃)₂, -CH₂CH₂-CO-CH₃, -CH₂CH₂-CO-CH₂CH₃, -CH₂CH₂-CO-CH(CH₃)₂, -CH₂-C(O)OCH₃, -CH₂-C(O)OCH₂CH₃, -CH₂-C(O)OCH(CH₃)₂, -CH₂CH₂-C(O)OCH₃, -CH₂CH₂-C(O)OCH₂CH₃, -CH₂CH₂-C(O)OCH(CH₃)₂, -CH₂-CO-CF₃, -CH₂-CO-CCl₃, -CH₂-CO-CH₂CF₃, -CH₂-CO-CH₂CCl₃, -CH₂CH₂-CO-CH₂CF₃, -CH₂CH₂-CO-CH₂CCl₃, -CH₂-C(O)OCH₂CF₃, -CH₂-C(O)OCF₂CF₃, -CH₂-C(O)OCH₂CCl₃, -CH₂-C(O)OCCl₂CCl₃, -CH₂CH₂-C(O)OCH₂CF₃, -CH₂CH₂-C(O)OCF₂CF₃, -CH₂CH₂-C(O)OCH₂CCl₃, -CH₂CH₂-C(O)O-CCl₂CCl₃; -COR⁶, -CONR⁷R⁸ oder -CH₂NR⁹R¹⁰.
- R⁵: steht ganz besonders bevorzugt für Wasserstoff; Methyl, Methoxymethyl, -CH₂-CHO, -CH₂CH₂-CHO, -CH₂-CO-CH₃, -CH₂-CO-CH₂CH₃, -CH₂-CO-CH(CH₃)₂ oder -COR⁶.

- R⁶: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen, -COR¹¹.
- R⁶: steht besonders bevorzugt für Wasserstoff, Methyl, Ethyl, n- oder iso-Propyl, tert-Butyl, Methoxy, Ethoxy, iso-Propoxy, tert-Butoxy, Cyclopropyl; Trifluormethyl, Trifluormethoxy, -COR¹¹.
- R⁶: steht ganz besonders bevorzugt für Wasserstoff, -COCH₃, -CHO, -COCH₂OCH₃, -COCO₂CH₃, -COCO₂CH₂CH₃.

- R⁷ und R⁸: stehen unabhängig voneinander bevorzugt für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁷ und R⁸: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹² enthalten kann.
- R⁷ und R⁸: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁷ und R⁸: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹² substituiert sein kann.
- R⁹ und R¹⁰: stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen.
- R⁹ und R¹⁰: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹² enthalten kann.
- R⁹ und R¹⁰: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁹ und R¹⁰: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹² substituiert sein kann.

- R¹¹: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R¹¹: steht besonders bevorzugt für Wasserstoff, Methyl, Ethyl, n- oder iso-Propyl, tert-Butyl, Methoxy, Ethoxy, iso-Propoxy, tert-Butoxy, Cyclopropyl; Trifluormethyl, Trifluormethoxy.

- R¹²: steht bevorzugt für Wasserstoff oder C₁-C₄-Alkyl.
- R¹²: steht besonders bevorzugt für Wasserstoff, Methyl, Ethyl, n- oder iso-Propyl, n-, iso-, sec- oder tert-Butyl.

- Z: steht bevorzugt für Z¹.
- Z¹: steht bevorzugt für einfach bis fünffach, gleich oder verschieden substituiertes Phenyl, wobei die Substituenten aus der Liste W¹ ausgewählt sind.
- Z¹: steht besonders bevorzugt für einfach substituiertes Phenyl, wobei die Substituenten aus der Liste W¹ ausgewählt sind.
- Z¹: steht auch besonders bevorzugt für zweifach, gleich oder verschieden substituiertes Phenyl, wobei die Substituenten aus der Liste W¹ ausgewählt sind.
- Z¹: steht auch besonders bevorzugt für dreifach, gleich oder verschieden substituiertes Phenyl, wobei die Substituenten aus der Liste W¹ ausgewählt sind.
- Z¹: steht ganz besonders bevorzugt für einfach in 4-Position substituiertes Phenyl, wobei die Substituenten aus der Liste W¹ ausgewählt sind.
- Z¹: steht ganz besonders bevorzugt für zweifach, gleich oder verschieden in 3,4-Position substituiertes Phenyl, wobei die Substituenten aus der Liste W¹ ausgewählt sind.
- Z¹: steht ganz besonders bevorzugt für zweifach, gleich oder verschieden in 2,4-Position substituiertes Phenyl, wobei die Substituenten aus der Liste W¹ ausgewählt sind.
- Z¹: steht ganz besonders bevorzugt für zweifach, gleich oder verschieden in 3,5-postition substituiertes Phenyl, wobei die Substituenten aus der Liste W¹ ausgewählt sind.
- Z¹: steht ganz besonders bevorzugt für dreifach, gleich oder verschieden in 2,4,6-Position substituiertes Phenyl, wobei die Substituenten aus der Liste W¹ ausgewählt sind.

- W¹: steht für Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy mit 1 bis 6 Kohlenstoffatomen in den jeweiligen Kohlenwasserstoffketten, Alkenylcarbonyl oder Alkinylcarbonyl, mit 2 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 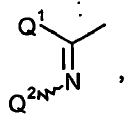 worin
Q¹ für Wasserstoff, Hydroxy oder Alkyl mit 1 bis 4 Kohlenstoffatomen, Halogenalkyl mit 1 bis 4 Kohlenstoffatomen und 1 bis 9 Fluor-, Chlor- und/oder Bromatomen oder Cycloalkyl mit 1 bis 6 Kohlenstoffatomen steht und
Q² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder. Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen.
- W¹: steht bevorzugt für Fluor, Chlor, Brom, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, jeweils zweifach verknüpftes Difluormethylendioxy oder Tetrafluorethylendioxy,
oder eine Gruppierung 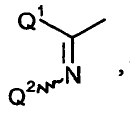 worin
Q¹ für Wasserstoff, Methyl, Ethyl oder Trifluormethyl steht und
Q² für Hydroxy, Methoxy, Ethoxy, Propoxy oder Isopropoxy steht.

- Z: steht auch bevorzugt für Z².
- Z²: steht bevorzugt für jeweils gegebenenfalls einfach bis vierfach, gleich oder verschieden durch Halogen und/oder C₁-C₄-Alkyl substituiertes Cycloalkyl oder Bicycloalkyl mit jeweils 3 bis 10 Kohlenstoffatomen.
- Z²: steht besonders bevorzugt für jeweils gegebenenfalls einfach bis vierfach, gleich oder verschieden durch Chlor und/oder Methyl substituiertes Cyclopropyl, Cyclopentyl, Cyclohexyl, Cycloheptyl, Cyclooctyl, Cyclononyl, Bicyclo[2.2.1]heptyl oder Bicyclo[2.2.2]octyl.
- Z²: steht ganz besonders bevorzugt für durch Chlor und Methyl substituiertes Cyclopropyl.

- Z: steht auch bevorzugt für Z³.
- Z³: steht bevorzugt für unsubstituiertes C₄-C₂₀-Alkyl oder für einfach oder mehrfach, gleich oder verschieden durch Fluor, Chlor, Brom, Iod und/oder C₃-C₆-Cycloalkyl substituiertes C₁-C₂₀-Alkyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- Z³: steht besonders bevorzugt für unsubstituiertes C₄-C₂₀-Alkyl.
- Z³: steht auch besonders bevorzugt für durch Chlor, Cyclopropyl, Dichlorcyclopropyl, Cyclobutyl, Cyclopentyl oder Cyclohexyl substituiertes C₄-C₂₀-Alkyl.

- Z: steht auch bevorzugt für Z⁴.
- Z⁴: steht bevorzugt für jeweils gegebenenfalls einfach oder mehrfach, gleich oder verschieden durch Fluor, Chlor, Brom, Iod und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- Z⁴: steht besonders bevorzugt für C₂-C₂₀-Alkenyl oder C₂-C₂₀-Alkinyl.

- Z und R⁴: stehen auch bevorzugt gemeinsam mit den Kohlenstoffatomen, an die sie gebunden sind, für einen gegebenenfalls einfach bis vierfach, gleich oder verschieden substituierten 5- oder 6-gliedrigen carbocyclischen oder heterocyclischen Ring.
- Z und R⁴: stehen auch besonders bevorzugt gemeinsam mit den Kohlenstoffatomen, an die sie gebunden sind, für einen gegebenenfalls einfach, zweifach oder dreifach durch Methyl substituierten 5- oder 6-gliedrigen carbocyclischen Ring.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Bevorzugt sind außerdem Verbindungen der Formel (Ic) 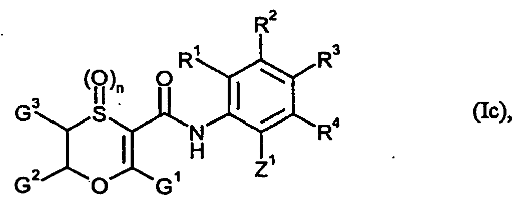 in welcher
- G¹, G², G³, n, R¹, R², R³, R⁴ und Z¹: die oben angegebenen Bedeutungen haben.

Besonders bevorzugt sind Verbindungen der Formel (Ic), in welcher G¹ für Trifluormethyl steht.

Besonders bevorzugt sind Verbindungen der Formel (Ic), in welcher G¹ für Difluormethyl steht.

Besonders bevorzugt sind Verbindungen der Formel (Ic), in welcher R¹, R², R³ und R⁴ jeweils für Wasserstoff stehen.

Besonders bevorzugt sind Verbindungen der Formel (Ic), in welcher n für 0 steht.

Bevorzugt sind außerdem Verbindungen der Formel (Id) 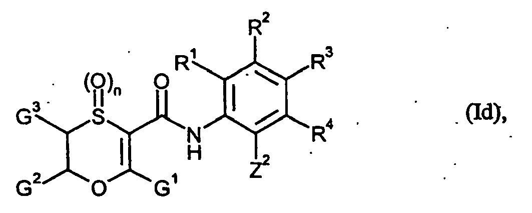 in welcher
- G¹, G², G³, n, R¹, R², R³, R⁴ und Z²: die oben angegebenen Bedeutungen haben.

Besonders bevorzugt sind Verbindungen der Formel (Id), in welcher G¹ für Trifluormethyl steht.

Besonders bevorzugt sind Verbindungen der Formel (Id), in welcher G¹ für Difluormethyl steht.

Besonders bevorzugt sind Verbindungen der Formel (Id), in welcher R¹, R², R³ und R⁴ jeweils für Wasserstoff stehen.

Besonders bevorzugt sind Verbindungen der Formel (Id), in welcher n für 0 steht

Bevorzugt sind außerdem Verbindungen der Formel (Ie) 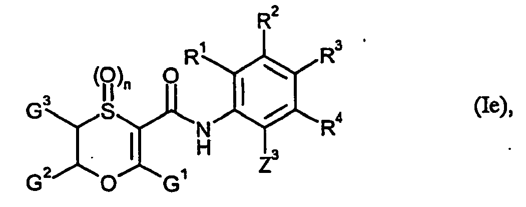 in welcher
- G¹, G², G³, n, R¹, R², R³, R⁴ und Z³: die oben angegebenen Bedeutungen haben.

Besonders bevorzugt sind Verbindungen der Formel (Ie), in welcher G¹ für Trifluormethyl steht.

Besonders bevorzugt sind Verbindungen der Formel (Ie), in welcher G¹ für Difluormethyl steht.

Besonders bevorzugt sind Verbindungen der Formel (Ie), in welcher R¹, R², R³ und R⁴ jeweils für Wasserstoff stehen.

Besonders bevorzugt sind Verbindungen der Formel (Ie), in welcher n für 0 steht.

Bevorzugt sind außerdem Verbindungen der Formel (If) 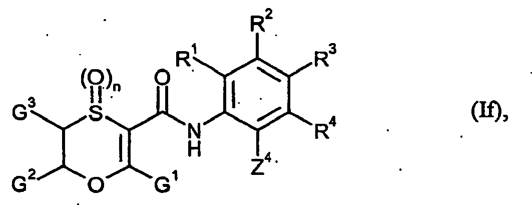 in welcher
- G¹, G², G³, n, R¹, R², R³, R⁴ und Z⁴: die oben angegebenen Bedeutungen haben.

Besonders bevorzugt sind Verbindungen der Formel (If), in welcher G¹ für Trifluormethyl steht.

Besonders bevorzugt sind Verbindungen der Formel (If), in welcher G¹ für Difluormethyl steht.

Besonders bevorzugt sind Verbindungen der Formel (If), in welcher R¹, R², R³ und R⁴ jeweils für Wasserstoff stehen.

Besonders bevorzugt sind Verbindungen der Formel (If), in welcher n für 0 steht.

Bevorzugt sind außerdem Verbindungen der Formel (Ig) 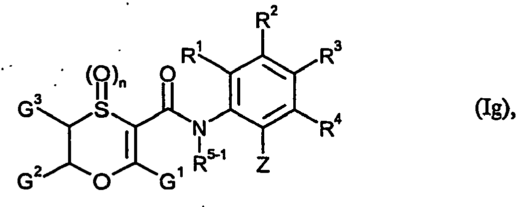 in welcher
- G¹, G², G³, n, R¹, R², R³, R⁴, R⁵⁻¹ und Z: die oben angegebenen Bedeutungen haben.
- R⁵⁻¹: steht bevorzugt für C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁶, -CONR⁷R⁸ oder -CH₂NR⁹R¹⁰.
- R⁵⁻¹: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl, -CH₂-CHO, -CH₂CH₂-CHO, -CH₂-CO-CH₃, -CH₂-CO-CH₂CH₃, -CH₂-CO-CH(CH₃)₂, -CH₂CH₂-CO-CH₃, -CH₂CH₂-CO-CH₂CH₃, -CH₂CH₂-CO-CH(CH₃)₂, -CH₂-C(O)OCH₃, -CH₂-C(O)OCH₂CH₃, -CH₂-C(O)OCH(CH₃)₂, -CH₂CH₂-C(O)OCH₃, -CH₂CH₂-C(O)OCH₂CH₃, -CH₂CH₂-C(O)OCH(CH₃)₂, -CH₂-CO-CF₃, -CH₂-CO-CCl₃, -CH₂-CO-CH₂CF₃, -CH₂-CO-CH₂CCl₃, -CH₂CH₂-CO-CH₂CF₃, -CH₂CH₂-CO-CH₂CCl₃, -CH₂-C(O)OCH₂CF₃, -CH₂-C(O)OCF₂CF₃, -CH₂-C(O)OCH₂CCl₃, -CH₂-C(O)OCCl₂CCl₃, -CH₂CH₂-C(O)OCH₂CF₃, -CH₂CH₂-C(O)OCF₂CF₃, -CH₂CH₂-C(O)OCH₂CCl₃, -CH₂CH₂-C(O)O-CCl₂CCl₃; -COR⁶, -CONR⁷R⁸ oder -CH₂NR⁹R¹⁰.
- R⁵⁻¹: steht ganz besonders bevorzugt für Methyl, Methoxymethyl, -CH₂-CHO, -CH₂CH₂-CHO, -CH₂-CO-CH₃, -CH₂-CO-CH₂CH₃, -CH₂-CO-CH(CH₃)₂ oder -COR⁶_{.}

Besonders bevorzugt sind Verbindungen der Formel (Ig), in welcher G¹ für Trifluormethyl steht.

Besonders bevorzugt sind Verbindungen der Formel (Ig), in welcher G¹ für Difluormethyl steht.

Besonders bevorzugt sind Verbindungen der Formel (Ig), in welcher R¹, R², R³ und R⁴ jeweils für Wasserstoff stehen.

Besonders bevorzugt sind Verbindungen der Formel (Ig), in welcher n für 0 steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Die Definition C₄-C₂₀-Alkyl umfasst den größten hierin definierten Bereich für einen Alkylrest. Im Einzelnen umfasst diese Definition die Bedeutungen n-, iso-, sec-, tert-Butyl, sowie jeweils alle isomeren Pentyle, Hexyle, Heptyle, Octyle, Nonyle, Decyle, Undecyle, Dodecyle, Tridecyle, Tetradecyle, Pentadecyle, Hexadecyle, Heptadecyle, Octadecyle, Nonadecyle und Eicosyle.

Die Definition C₂-C₂₀-Alkenyl umfasst den größten hierin definierten Bereich für einen Alkenylrest. Im Einzelnen umfasst diese Definition die Bedeutungen Ethenyl, n-, iso-Propenyl, n-, iso-, sec-, tert-Butenyl, sowie jeweils alle isomeren Pentenyle, Hexenyle, Heptenyle, Octenyle, Nonenyle, Decenyle, Undecenyle, Dodecenyle, Tridecenyle, Tetradecenyle, Pentadecenyle, Hexadecenyle, Heptadecenyle, Octadecenyle, Nonadecenyle und Eicosenyle.

Die Definition C₂-C₂₀-Alkinyl umfasst den größten hierin definierten Bereich für einen Alkinylrest. Im Einzelnen umfasst diese Definition die Bedeutungen Ethinyl, n-, iso-Propinyl, n-, iso-, sec-, tert-Butinyl, sowie jeweils alle isomeren Pentinyle, Hexinyle, Heptinyle, Octinyle, Noninyle, Decinyle, Undecinyle, Dodecinyle, Tridecinyle, Tetradecinyle, Pentadecinyle, Hexadecinyle, Heptadecinyle, Octadecinyle, Nonadecinyle und Eicosinyle.

Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenallyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefnitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend.

### Erläuterungen der Verfahren und Zwischenprodukte:

### Verfahren (a)

Verwendet man 6-Trifluormethyl-2,3-dihydro-1,4-oxathiin-5-carbonsäurechlorid und 4'-Chlor-2'-fluor-1,1'-biphenyl-2-amin als Ausgangsstoffe, so kann der Verlauf des erfindungsgemäßen Verfahrens (a) durch das folgende Formelschema veranschaulicht werden.

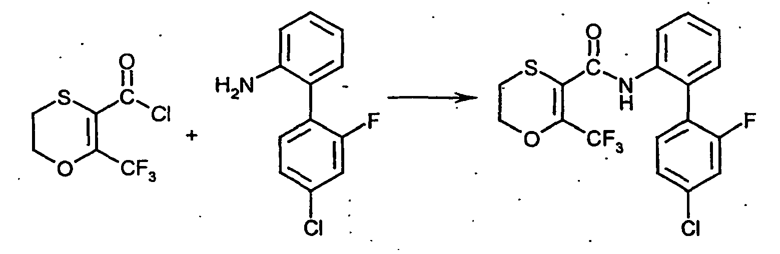

Die zur Durchführung des erfindungsgemäßen Verfahrens (a) als Ausgangsstoffe benötigten Oxathiincarbonsäure-Derivate sind durch die Formel (II) allgemein definiert. In dieser Formel (II) haben G¹, G², G³ und n bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für G¹, G², G³ und n angegeben wurden. X¹ steht bevorzugt für Chlor oder Hydroxy.

Die Ausgangsstoffe der Formel (II) sind größtenteils bekannt und/oder können nach bekannten Verfahren hergestellt werden (vgl. Han'guk Nonghwa Hakhoechi 2001, 44, 191-196). Neue Verbindungen der Formel (II) sowie Verfahren zu deren Herstellung sind den Herstellungsbeispielen zu entnehmen.

Verbindungen der Formel (II), in welcher n für 1 oder 2 steht, werden aus den entsprechenden Verbindungen, in welchen n für 0 steht, durch übliche Methoden der Oxidation erhalten, beispielsweise durch Umsetzung mit Wasserstoffperoxid (H₂O₂) in Gegenwart von Ameisensäure und einem Verdünnungsmittel (z.B. 4-Methyl-2-pentanon). Der Grad der Oxidation lässt sich jeweils durch die Reaktionsbedingungen steuern.

Auf die gleiche Weise ist es auch möglich, Verbindungen der Formel (I), in welcher n für 0 steht, zu oxidieren und so Verbindungen der Formel (I) zu erhalten, in welcher n für 1 oder 2 steht. Der Grad der Oxidation lässt sich jeweils durch die Reaktionsbedingungen steuern.

Die zur Durchführung des erfindungsgemäßen Verfahrens (a) weiterhin als Ausgangsstoffe benötigten Anilin-Derivate sind durch die Formel (III) allgemein definiert. In dieser Formel (III) haben R¹, R², R³, R⁴, R⁵ und Z bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Ausgangsstoffe der Formel (III) sind größtenteils bekannt und/oder können nach bekannten Verfahren hergestellt werden (vgl. z.B. Bull. Korean Chem. Soc. 2000, 21, 165-166; Chem. Pharm. Bull. 1992, 40, 240-244; Heterocycles 1989, 29, 1013-1016; J. Med. Chem. 1996, 39, 892-903; Synthesis 1995, 713-16; Synth. Commun. 1994, 24, 267-272; Synthesis 1994, 42-144; DE-A 27 27 416; EP-A 0 824 099; WO 93/11117, EP-A 0 545 099, EP-A 0 589 301, EP-A 0 589 313 und WO 02/38542).

Es ist auch möglich, zunächst Anilin-Derivate der Formel (III), in welcher R⁵ für Wasserstoff steht, herzustellen und die so erhaltenen Verbindungen anschließend nach üblichen Methoden zu derivatisieren [z.B. analog zum erfindungsgemäßen Verfahren (i)].

Neue Verbindungen der Formel (III) sowie Verfahren zu deren Herstellung sind den Herstellungsbeispielen zu entnehmen.

### Verfahren (b)

Verwendet man *N*-(2-Bromphenyl)-6-trifluormethyl-2,3-dihydro-1,4-oxathiin-5-carboxamid und 4-Chlor-2-fluorphenylboronsäure als Ausgangsstoffe sowie einen Katalysator, so kann der Verlauf des erfindungsgemäßen Verfahrens (b) durch das folgende Formelschema veranschaulicht werden.

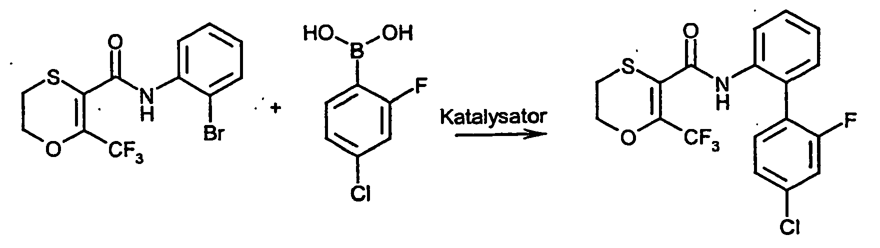

Die zur Durchführung des erfindungsgemäßen Verfahrens (b) als Ausgangsstoffe benötigten Halogenoxathiincarboxamide sind durch die Formel (IV) allgemein definiert. In dieser Formel (IV) haben G¹, G², G³, n, R¹, R², R³, R⁴ und R⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste bzw. diesen Index angegeben wurden. X² steht für Brom oder Iod.

Die Halogenoxathiincarboxamide der Formel (IV) sind noch nicht bekannt. Sie sind als neue chemische Verbindungen ebenfalls Gegenstand der vorliegenden Anmeldung. Sie werden erhalten, indem man
j) Oxathiincarbonsäure-Derivate der Formel (II) 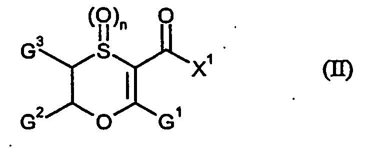 in welcher
   - G¹, G², G³: und n die oben angegebenen Bedeutungen haben und
   - X¹: für Halogen oder Hydroxy steht,
   mit Halogenanilinen der Formel (XIV), 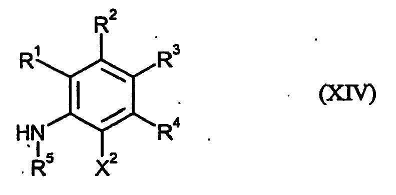 in welcher
   - R¹, R², R³, R⁴, R⁵ und X²: die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (j)

Verwendet man 6-Trifluormethyl-2,3-dihydro-1,4-oxathiin-5-carbonsäurechlorid und 2-Bromanilin als Ausgangsstoffe, so kann der Verlauf des erfindungsgemäßen Verfahrens (j) durch das folgende Formelschema veranschaulicht werden.

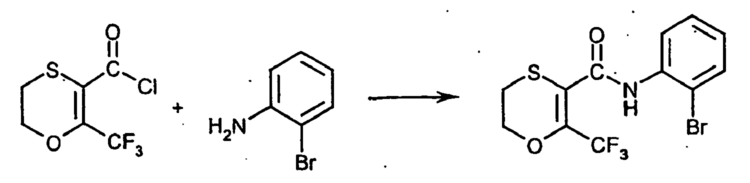

Die zur Durchführung des erfindungsgemäßen Verfahrens (j) als Ausgangsstoffe benötigten Oxathiincarbonsäure-Halogide der Formel (II) sind bereits weiter oben im Zusammenhang mit dem erfindungsgemäßen Verfahren (a) beschrieben worden.

Die zur Durchführung des erfindungsgemäßen Verfahrens (j) weiterhin als Ausgangsstoffe benötigten Halogenaniline sind durch die Formel (XIV) allgemein definiert. In dieser Formel (XIV) haben R¹, R², R³, R⁴, R⁵ und X² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I), bzw. die Vorprodukte der Formel (III) als bevorzugt, besonders bevorzugt bzw. ganz besonders bevorzugt für diese Reste angegeben wurden.

Die Halogenaniline der Formel (XIV) sind handelsübliche Synthesechemikalien oder können nach bekannten Verfahren erhalten werden. Im Fall, dass R⁵ nicht für Wasserstoff steht, kann der Rest R⁵ auf der Stufe der Verbindungen der Formel (XIV) durch übliche Derivatisierungsmethoden eingeführt werden. Es ist auch möglich, zunächst Verbindungen der Formel (IV), in denen R⁵ für Wasserstoff steht, herzustellen und die erhaltenen Produkte anschließend durch übliche Methoden zu derivatisieren [vgl. das erfindungsgemäße Verfahren (i)].

Die zur Durchführung des erfindungsgemäßen Verfahrens (b) weiterhin als Ausgangsstoffe benötigten Boronsäure-Derivate sind durch die Formel (V) allgemein definiert. In dieser Formel (V) hat Z¹ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für Z¹ angegeben wurden. A¹ und A² stehen jeweils für Wasserstoff oder zusammen für Tetramethylethylen.

Die Boronsäure-Derivate der Formel (V) sind bekannt und/oder lassen sich nach bekannten Verfahren herstellen (vgl. z.B. WO 01/90084 und US 5,633,218).

### Verfahren c)

Verwendet man 2-{[6-(Trifluormethyl)-2,3-dihydro-1,4-oxathiin-5-yl]carbonylamino}phenylboronsäure und 1-Brom-4-chlor-2-fluorbenzol als Ausgangsstoffe sowie einen Katalysator, so kann der Verlauf des erfindungsgemäßen Verfahrens (c) durch das folgende Formelschema veranschaulicht werden.

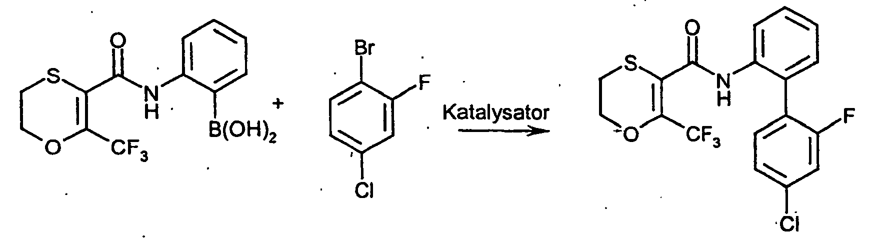

Die zur Durchführung des erfindungsgemäßen Verfahrens (c) als Ausgangsstoffe benötigten Oxathiincarboxamid-Boronsäure-Derivate sind durch die Formel (VI) allgemein definiert. In dieser Formel (VI) haben G¹, G², G³, n, R¹, R², R³, R⁴ und R⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste bzw. diesen Index angegeben wurden. A³ und A⁴ stehen jeweils für Wasserstoff oder zusammen für Tetramethylethylen.

Die Oxathiincarboxamid-Boronsäure-Derivate der Formel (VI) sind noch nicht bekannt. Sie sind neue chemische Verbindungen und ebenfalls Gegenstand der vorliegenden Anmeldung. Sie werden erhalten, indem man
k) ein Oxathiincarbonsäurederivat der Formel (II) 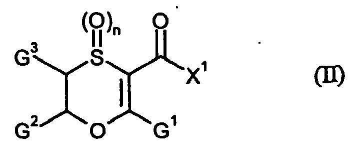 in welcher
   - G¹, G², G³: und n die oben angegebenen Bedeutungen haben und
   - X¹: für Halogen oder Hydroxy steht,
   mit einem Anilinboronsäurederivat der Formel (XV) 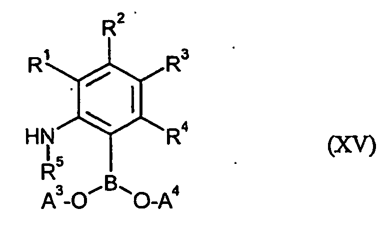 in welcher
   - R¹, R², R³, R⁴, R⁵, A³ und A⁴: die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (k)

Verwendet man 6-Trifluormethyl-2,3-dihydro-1,4-oxathiin-5-carbonsäurechlorid und 2-Aminophenylboronsäure als Ausgangsstoffe, so kann der Verlauf des erfindungsgemäßen Verfahrens (k) durch das folgende Formelschema veranschaulicht werden.

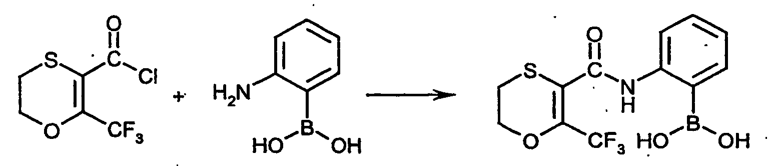

Die zur Durchführung des erfindungsgemäßen Verfahrens (k) als Ausgangsstoffe benötigten Oxathiincarbonsäurederivate der Formel (II) sind bereits weiter oben im Zusammenhang mit dem erfindungsgemäßen Verfahren (a) beschrieben worden.

Die weiterhin zur Durchführung des erfindungsgemäßen Verfahrens (k) als Ausgangsstoffe benötigten Anilinboronsäurederivate sind durch die Formel (XV) allgemein definiert. In dieser Formel (XV) haben R¹, R², R³, R⁴ und R⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. A³ und A⁴ stehen jeweils für Wasserstoff oder gemeinsam für Tetramethylethylen.

Die Anilinboronsäurederivate der Formel (XV) sind bekannte Synthesechemikalien oder können nach bekannten Verfahren erhalten werden. Im Fall, dass R⁵ nicht für Wasserstoff steht, kann der Rest R⁵ auf der Stufe der Verbindungen der Formel (XV) durch übliche Derivatisierungsmethoden eingeführt werden. Es ist auch möglich, zunächst Verbindungen der Formel (VI), in denen R⁵ für Wasserstoff steht, herzustellen und die erhaltenen Produkte anschließend durch übliche Methoden zu derivatisieren [vgl. das erfindungsgemäße Verfahren (i)].

Die zur Durchführung des erfindungsgemäßen Verfahrens (c) weiterhin als Ausgangsstoffe benötigten Phenyl-Derivate sind durch die Formel (VII) allgemein definiert. In dieser Formel (VII) hat Z¹ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für Z¹ angegeben wurden. X³ steht für Chlor, Brom, Iod oder Trifluormethylsulfonat.

Die Phenyl-Derivate der Formel (VII) sind bekannte Synthesechemikalien.

### Verfahren d)

Verwendet man *N*-(2-Bromphenyl)-6-trifluormethyl-2,3-dihydro-1,4-oxathiin-5-carboxamid und 1-Brom-4-chlor-2-fluorbenzol als Ausgangsstoffe sowie einen Katalysator und 4,4,4',4',5,5,5',5'-Octamethyl-2,2'-bis-1,3,2-dioxaborolan, so kann der Verlauf des erfindungsgemäßen Verfahrens (d) durch das folgende Formelschema veranschaulicht werden.

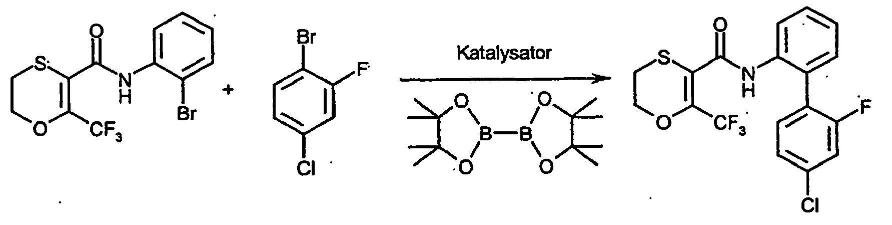

Die zur Durchführung des erfindungsgemäßen Verfahrens (d) als Ausgangsstoffe benötigten Halogenoxathiincarboxmide der Formel (IV), sowie die Phenyl-Derivate der Formel (VII) sind bereits weiter oben im Zusammenhang mit den erfindungsgemäßen Verfahren (b) und (c) beschrieben worden.

Das weiterhin zur Durchführung des erfindungsgemäßen Verfahrens (d) benötigte 4,4,4',4',5,5,5',5'-Octamethyl-2,2'-bis-1,3,2-dioxaborolan ist eine handelsübliche Synthesechemikalie.

### Verfahren e)

Hydriert man beispielsweise *N*-[2-(1,3-Dimethylbut-1-enyl)phenyl][6-(trifluormethyl)(2,3-dihydro-1,4-oxathiin-5-yl)]carboxamid, so kann der Verlauf des erfindungsgemäßen Verfahrens (e) durch das folgende Formelschema veranschaulicht werden.

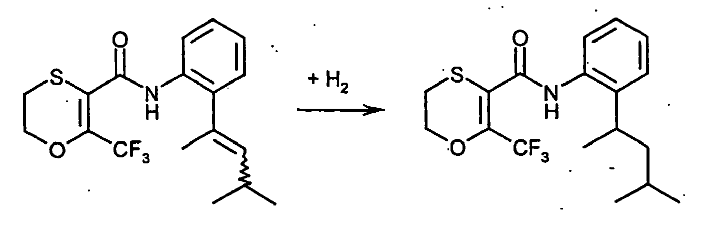

Die zur Durchführung des erfindungsgemäßen Verfahrens (e) als Ausgangsstoffe benötigten Oxathiincarboxamide sind durch die Formel (Ia) allgemein definiert. In dieser Formel (Ia) haben G¹, G², G³, n, R¹, R², R³ und R⁴ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste bzw. diesen Index angegeben wurden.
- X⁴: steht bevorzugt für jeweils gegebenenfalls einfach oder mehrfach, gleich oder verschieden durch Fluor, Chlor, Brom, Iod und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- X⁴: steht besonders bevorzugt für C₂-C₂₀-Alkenyl oder C₂-C₂₀-Alkinyl.

Die Verbindungen der Formel (Ia) sind erfindungsgemäße Verbindungen und können nach den Verfahren (a), (f), (g) oder (h) hergestellt werden.

### Verfahren (f)

Dehydratisiert man beispielsweise *N*-[2-(1-Hydroxy-1,3-dimethylbutyl)phenyl][6-trifluormethyl)(2,3-dihydro-1,4-oxathiin-5-yl)]carboxamid, so kann der Verlauf des erfindungsgemäßen Verfahrens (f) durch das folgende Formelschema veranschaulicht werden.

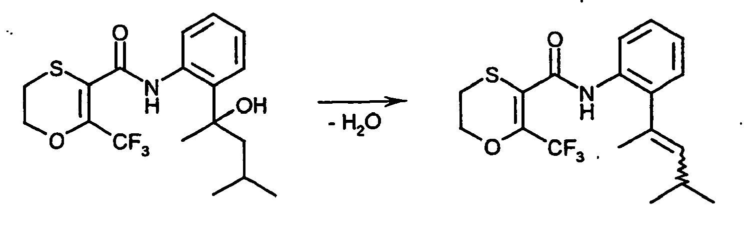

Die zur Durchführung des erfindungsgemäßen Verfahrens (f) als Ausgangsstoffe benötigten Hydroxyalkyloxathiincarboxamide sind durch die Formel (VIII) allgemein definiert. In dieser Formel (VIII) haben G¹, G², G³, n, R¹, R², R³, R⁴ und R⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste bzw. diesen Index angegeben wurden.
- X⁵: steht bevorzugt für gegebenenfalls zusätzlich einfach bis vierfach, gleich oder verschieden durch Chlor, Fluor, Brom und/oder C₃-C₆-Cycloalkyl substituiertes C₂-C₁₂- Hydroxyalkyl, wobei der Cycloalkylteil seinerseits gegebenenfalls durch Halogen und/oder C₁-C₄-Alkyl substituiert sein kann.
- X⁵: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Hydroxyethyl, Hydroxypropyl, Hydroxybutyl, Hydroxypentyl, Hydroxyhexyl, Hydroxyheptyl, Hydroxyoctyl, Hydroxynonyl oder Hydroxydecyl.

Die Verbindungen der Formel (VIII) sind noch nicht bekannt und als neue Verbindungen ebenfalls Gegenstand der vorliegenden Anmeldung:

Es wurde auch gefunden, dass die Hydroxyalkylpyrazolylcarboxamide der Formel (VIII) sehr gute mikrobizide Eigenschaften besitzen und zur Bekämpfung unerwünschter Mikroorganismen sowohl im Pflanzenschutz als auch im Materialschutz eingesetzt werden können.

Die Hydroxyalkylpyrazolylcarboxamide der Formel (VIII) werden erhalten, indem man
l) Oxathiincarbonsäurederivate der Formel (II) 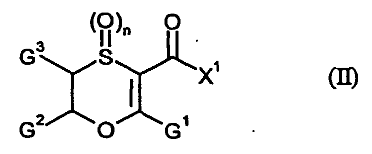 in welcher
   - G¹, G², G³: und n die oben angegebenen Bedeutungen haben und
   - X¹: für Halogen oder Hydroxy steht,
   mit einem Hydroxyalkylanilinderivat der Formel (XVI) 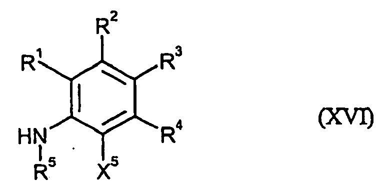 in welcher
   - R¹, R², R³, R⁴, R⁵ und X⁵: die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (I)

Verwendet man beispielsweise 6-Trifluormethyl-2,3-dihydro-1,4-oxathiin-5-carbonsäurechlorid und 2-(2-Aminophenyl)-2-heptanol als Ausgangsstoffe, so kann der Verlauf des erfindungsgemäßen Verfahrens (1) durch das folgende Formelschema veranschaulicht werden:

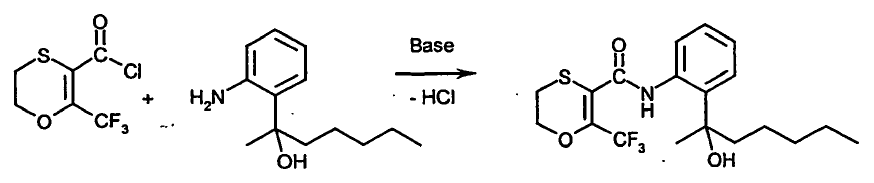

Die zur Durchführung des erfindungsgemäßen Verfahrens (1) als Ausgangsstoffe benötigten Oxathiincarbonsäurederivate der Formel (II) sind bereits weiter oben im Zusammenhang mit dem erfindungsgemäßen Verfahren (a) beschrieben worden.

Die zur Durchführung des erfindungsgemäßen Verfahrens (l) als Ausgangsstoffe weiterhin benötigten Hydroxyalkylanilinderivate sind durch die Formel (XVI) allgemein definiert. In dieser Formel (XVI) haben R¹, R², R³, R⁴, R⁵ und X⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formeln (I) bzw. (VIII) als bevorzugt, besonders bevorzugt bzw. ganz besonders bevorzugt für diese Reste angegeben wurden.

Die Hydroxyalkylanilinderivate der Formel (XVI) sind bekannt und/oder können nach bekannten Methoden erhalten werden (vgl. z.B. US 3,917,592 oder EP-A 0 824 099). Im Fall, dass R⁵ nicht für Wasserstoff steht, kann der Rest R⁵ auf der Stufe der Verbindungen der Formel (XVI) durch übliche Derivatisierungsmethoden eingeführt werden. Es ist auch möglich, zunächst Verbindungen der Formel (VIII), in denen R⁵ für Wasserstoff steht, herzustellen und die erhaltenen Produkte anschließend durch übliche Methoden zu derivatisieren [vgl. das erfindungsgemäße Verfahren (i)].

### Verfahren (g)

Verwendet man beispielsweise *N*-(2-Bromphenyl)-6-trifluormethyl-2,3-dihydro-1,4-oxathiin-5-carboxamid und 1-Hexin als Ausgangsstoffe sowie einen Katalysator, so kann der Verlauf des erfindungsgemäßen Verfahrens (g) durch das folgende Formelschema veranschaulicht werden.

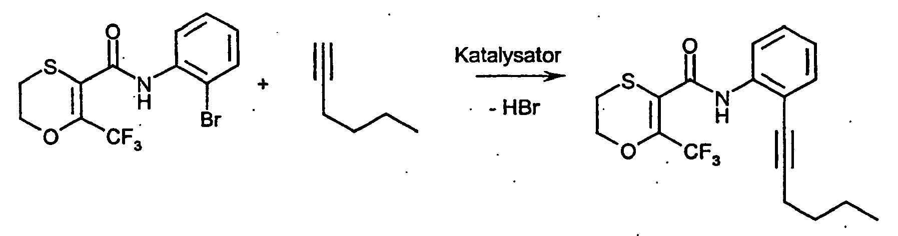

Die zur Durchführung des erfindungsgemäßen Verfahrens (g) als Ausgangsstoffe benötigten Halogenoxathiincarboxamide der Formel (IV) sind bereits weiter oben im Zusammenhang mit dem erfindungsgemäßen Verfahrens (c) beschrieben worden.

Die zur Durchführung des erfindungsgemäßen Verfahrens (g) weiterhin als Ausgangsstoffe benötigten Alkine sind durch die Formel (IX) allgemein definiert.
- A⁵: steht bevorzugt für gegebenenfalls einfach bis vierfach, gleich oder verschieden durch Fluor, Chlor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- A⁵: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Alkine der Formel (VI) sind bekannte Synthesechemikalien.

Die zur Durchführung des erfindungsgemäßen Verfahrens (g) weiterhin alternativ als Ausgangsstoffe benötigten Alkene sind durch die Formel (X) allgemein definiert.
- A⁶, A⁷ und A⁸: stehen unabhängig voneinander bevorzugt jeweils für Wasserstoff oder gegebenenfalls einfach bis vierfach, gleich oder verschieden durch Fluor, Chlor, Brom und/oder C₃-C₆-Cycloalkyl substituiertes Alkyl, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 12 nicht übersteigt.
- A⁶, A⁷ und A⁸: stehen unabhängig voneinander besonders bevorzugt jeweils für Wasserstoff oder gegebenenfalls einfach bis vierfach, gleich oder verschieden durch Fluor, Cyclopropyl, Difluorcyclopropyl, Cyclobutyl, Cyclopentyl und/ oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl, wobei die Gesamtzahl der Kohlenstoffatome des offenkettigen Molekülteils die Zahl 12 nicht übersteigt.

Die Alkene der Formel (VII) sind bekannte Synthesechemikalien.

### Verfahren (h)

Verwendet man *N*-(2-Acetylphenyl)[6-(trifluormethyl)(2,3-dihydro-1,4-oxathiin-5-yl)]-carboxamid und Butyl(triphenyl)-phosphonium-iodid als Ausgangsstoffe, so kann der Verlauf des erfindungsgemäßen Verfahrens (h) durch das folgende Formelschema veranschaulicht werden:

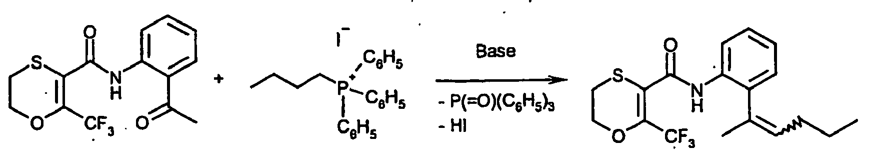

Die zur Durchführung des erfindungsgemäßen Verfahrens (h) als Ausgangsstoffe benötigten Ketone sind durch die Formel (XI) allgemein definiert. In dieser Formel haben G¹, G², G³, n, R¹, R², R³, R⁴ und R⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste bzw. diesen Index angegeben wurden.
- A⁹: steht bevorzugt für gegebenenfalls einfach bis vierfach, gleich oder verschieden durch Fluor, Chlor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- A⁹: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Ketone der Formel (XI) sind noch nicht bekannt. Sie sind als neue chemische Verbindungen ebenfalls Gegenstand der vorliegenden Anmeldung. Sie werden erhalten, indem man
m) Oxathiincarbonsäurederivate der Formel (II) 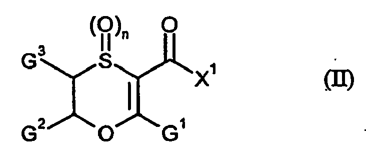 in welcher
   - G¹, G², G³: und n die oben angegebenen Bedeutungen haben und
   - X¹: für Halogen oder Hydroxy steht,
   mit Ketoanilinen der Formel (XVII) 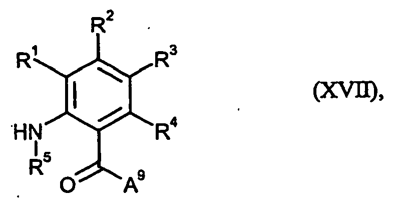 in welcher
   - R¹, R², R³, R⁴, R⁵ und A⁹: die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (m)

Verwendet man 6-Trifluornlethyl-2,3-dihydro-1,4-oxathiin-5-carbonsäurechlorid und 1-(2-Aminophenym)ethanon als Ausgangsstoffe, so kann der Verlauf des erfindungsgemäßen Verfahrens (m) durch das folgende Formelschema veranschaulicht werden:

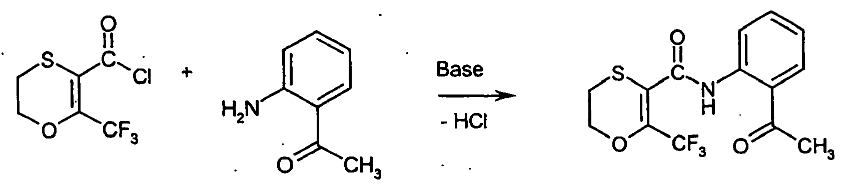

Die zur Durchführung des erfindungsgemäßen Verfahrens (m) als Ausgangsstoffe benötigten Oxathiincarbonsäurederivate der Formel (II) sind bereits weiter oben im Zusammenhang mit dem erfindungsgemäßen Verfahrens a) beschrieben worden.

Die zur Durchführung des erfindungsgemäßen Verfahrens (m) weiterhin als Ausgangsstoffe benötigten Ketoaniline sind durch die Formel (XVII) allgemein definiert. In dieser Formel (XVII) haben R¹, R², R³, R⁴, R⁵ und A⁹ bevorzugt, besonders bevorzugt bzw. ganz besonders bevorzugt diejenigen Bedeutungen, die bereits im Zusammenhang mit der Beschreibung der erfindungsgemäßen Verbindungen der Formeln (I) bzw. (XI) als bevorzugt, besonders bevorzugt bzw. ganz besonders bevorzugt für diese Reste angegeben wurden.

Die Ketoaniline der Formel (XII) sind allgemein übliche Synthesechemikalien (vgl. z.B. J. Am. Chem. Soc. 1978, 100, 4842-4857 oder US 4,032,573). Im Fall, dass R⁵ nicht für Wasserstoff steht, kann der Rest R⁵ auf der Stufe der Verbindungen der Formel (XVII) durch übliche Derivatisierungsmethoden eingeführt werden. Es ist auch möglich, zunächst Verbindungen der Formel (XI), in denen R⁵ für Wasserstoff steht, herzustellen und die erhaltenen Produkte anschließend durch übliche Methoden zu derivatisieren [vgl. das erfindungsgemäße Verfahren (i)].

Die zur Durchführung des erfindungsgemäßen Verfahrens (h) weiterhin als Ausgangsstoffe benötigten Phosphorverbindungen sind durch die Formel (XII) allgemein definiert.
- A¹⁰: steht bevorzugt für gegebenenfalls einfach bis vierfach, gleich oder verschieden durch Chlor, Fluor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- A¹⁰: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.
- Px: steht bevorzugt für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃.

Die Phosphorverbindungen der Formel (XII) sind bekannt und/oder können nach bekannten Verfahren hergestellt werden (vgl. z.B. Justus Liebigs Ann. Chem. 1953, 580, 44-57 oder Pure Appl. Chem. 1964, 9, 307-335).

### Verfahren (i)

Verwendet man *N-*[2-(1,3-Dimethylbutyl)phenyl]-2-(trifluormethyl)-5,6-dihydro-1,4-oxathiin-3-carboxamid und Acetylchlorid als Ausgangsstoffe, so kann der Verlauf des erfindungsgemäßen Verfahrens (i) durch das folgende Formelschema veranschaulicht werden:

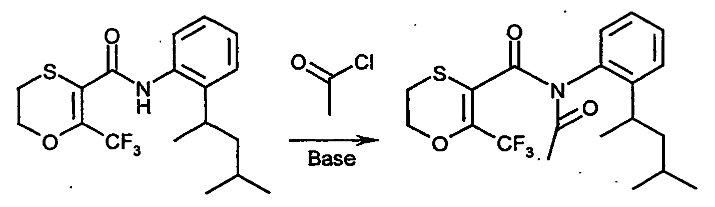

Die zur Durchführung des erfindungsgemäßen Verfahrens (i) als Ausgangsstoffe benötigten Iodpyrazolylcarboxanilide sind durch die Formel (Ib) allgemein definiert. In dieser Formel (Ib) haben G¹, G², G³, n, R¹, R², R³, R⁴ und Z bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste bzw. diesen Index angegeben wurden.

Die Verbindungen der Formel (Ib) sind erfindungsgemäße Verbindungen und können nach den Verfahren (a) bis (h) hergestellt werden.

Die zur Durchführung des erfindungsgemäßen Verfahrens (i) weiterhin als Ausgangsstoffe benötigten Halogenide sind durch die Formel (XIII) allgemein definiert. In dieser Formel (XIII) steht R⁵⁻¹ bevorzugt, besonders bevorzugt bzw. ganz besonders bevorzugt für diejenigen Bedeutungen, die bereits oben in Zusammenhang mit der Beschreibung der Verbindungen der Formel (Ig) als bevorzugt, besonders bevorzugt bzw. ganz besonders bevorzugt für diese Reste angegeben wurden. X⁶ steht für Chlor, Brom oder Iod.

Halogenide der Formel (XIII) sind bekannt.

### Reaktionsbedingungen

Als Verdünnungsmittel zur Durchführung der erfindungsgemäßen Verfahren (a), (j), (k), (1) und (m) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Die erfindungsgemäßen Verfahren (a), (j), (k), (l) und (m) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren (a), (j), (k), (l) und (m) werden gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N-Dicyclohexylcarbodiimid (DCC) oder andere übliche Kondensationsmittel, wie Phosphorpentoxid, Polyphosphorsäure, N.N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Die erfindungsgemäßen Verfahren (a), (j), (k), (l) und (m) werden gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung der erfindungsgemäßen Verfahren (a), (j), (k), (l) und (m) in einem größeren Bereich variiert werden. Im allgemeinen arbeitet man bei Temperaturen von 0°C bis 150°C; vorzugsweise bei Temperaturen von 0°C bis 80°C.

Zur Durchführung des erfindungsgemäßen Verfahrens (a) zur Herstellung der Verbindungen der Formel (I) setzt man pro Mol des Oxathiincarbonsäurederivates der Formel (II) im Allgemeinen 0,8 bis 15 Mol, vorzugsweise 0,8 bis 8 Mol an Anilin-Derivat der Formel (III) ein.

Zur Durchführung des erfindungsgemäßen Verfahrens (j) zur Herstellung der Verbindungen der Formel (IV) setzt man pro Mol des Oxathiincarbonsäurederivates der Formel (II) im Allgemeinen 0,8 bis 15 Mol, vorzugsweise 0,8 bis 8 Mol an Halogenaniline der Formel (XIV) ein.

Zur Durchführung des erfindungsgemäßen Verfahrens (k) zur Herstellung der Verbindungen der Formel (VI) setzt man pro Mol des Oxathiincarbonsäurederivates der Formel (II) im Allgemeinen 0,8 bis 15 Mol, vorzugsweise 0,8 bis 8 Mol an Anilinboronsäurederivat der Formel (XV) ein.

Zur Durchführung des erfindungsgemäßen Verfahrens (1) zur Herstellung der Verbindungen der Formel (VIII) setzt man pro Mol des Oxathiincarbonsäurederivates der Formel (II) im Allgemeinen 0,8 bis 15 Mol, vorzugsweise 0,8 bis 8 Mol an Hydroxyalkylanilinderivat der Formel (XVI) ein.

Zur Durchführung des erfindungsgemäßen Verfahrens (m) zur Herstellung der Verbindungen der Formel (IX) setzt man pro Mol des Oxathiincarbonsäurederivates der Formel (II) im Allgemeinen 0,8 bis 15 Mol, vorzugsweise 0,8 bis 8 Mol an Ketoanilin der Formel (XVII) ein.

Als Verdünnungsmittel zur Durchführung der erfindungsgemäßen Verfahren (b), (c) und (d) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahren (b), (c) und (d) in einem größeren Bereich variiert werden. Im allgemeinen arbeitet man bei Temperaturen von 0°C bis 180°C, vorzugsweise bei Temperaturen von 20°C bis 150°C.

Die der erfindungsgemäßen Verfahren (b), (c) und (d) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die der erfindungsgemäßen Verfahren (b), (c) und (d) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder (1,1'-Bis(diphenylphosphino)-ferrocenpalladiurn(II)chlorid) infrage.

Es kann auch ein Palladiumkomplex in der Reaktionsmischung erzeugt werden, wenn man ein Palladiumsalz und ein Komplexligand, wie z.B. Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-tolyl)-phosphan, Natrium 3-(Diphenylphosphino)benzolsulfonat, Tris-2-(Methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt

Zur Durchführung des erfindungsgemäßen Verfahrens (b) zur Herstellung der Verbindungen der Formel (I) setzt man pro Mol des Halogenoxathiincarboxamids der Formel (IV) im Allgemeinen 1 bis 15 Mol, vorzugsweise 2 bis 8 Mol an Boronsäurederivat der Formel (V) ein.

Zur Durchführung des erfindungsgemäßen Verfahrens (c) zur Herstellung der Verbindungen der Formel (I) setzt man pro Mol des Oxathiincarboxamid-Boronsäure-Derivate der Formel (VI) im Allgemeinen 0,8 bis 15 Mol, vorzugsweise 0,8 bis 8 Mol an Phenyl-Derivat der Formel (VII) ein.

Zur Durchführung des erfindungsgemäßen Verfahrens (d) zur Herstellung der Verbindungen der Formel (I) setzt man pro Mol des Halogenoxathiincarboxamides der Formel (IV) im Allgemeinen 0,8 bis 15 Mol, vorzugsweise 0,8 bis 8 Mol an Phenyl-Derivat der Formel (VII) und 0,8 bis 15 Mol, vorzugsweise 0,8 bis 8 Mol an 4,4,4',4,5,5,5',5'-Octamethyl-2,2'-bis-1,3,2-dioxaborolan ein.

Als Verdünnungsmittel zur Durchführung des erfindungsgemäßen Verfahrens (e) kommen alle inerten organischen Lösungsmittel in Betracht. Hierzu gehören vorzugsweise aliphatische oder alicyclische Kohlenwasserstoffe, wie beispielsweise Petrolether, Hexan, Heptan, Cyclohexan, Methylcyclohexan oder Decalin; Ether, wie Diethylether, Diisopropylether, Methyl-t-butylether, Methyl-t-Amylether, Dioxan, Tetrahydrofuran, 1,2- Dimethoxyethan oder 1,2-Diethoxyethan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße (e) Verfahren wird in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Reaktionstemperaturen können bei der Durchführung des erfindungsgemäßen Verfahrens (e) in einem größeren Bereich variiert werden. Im allgemeinen arbeitet man bei Temperaturen von 0°C bis 150°C, vorzugsweise bei Temperaturen von 20°C bis 100°C.

Als Verdünnungsmittel zur Durchführung des erfindungsgemäßen Verfahrens (f) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methylisobutylketon oder Cyclohexanon; Nitrile, wie Acetonitril; Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren (f) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des erfindungsgemäßen Verfahrens (f) in einem größeren Bereich variiert werden. Im allgemeinen arbeitet man bei Temperaturen von 0°C bis 150°C, vorzugsweise bei Temperaturen von 0°C bis 100°C.

Als Verdünnungsmittel zur Durchführung des erfindungsgemäßen Verfahrens (g) kommen alle inerten organischen Lösungsmittel in Betracht. Hierzu gehören vorzugsweise Nitrile, wie Acetonitril, Propionitril, n- oder i-Butyronitril oder Benzonitril oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (g) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfmdungsgemäße Verfahren (g) wird in Gegenwart eines oder mehrerer Katalysatoren durchgeführt.

Dazu eignen sich besonders Palladiumsalze oder -komplexe. Hierzu kommen vorzugsweise Palladiumchlorid, Palladiumacetat, Tetrakis-(triphenylphosphin)-Palladium oder Bis-(triphenylphosphin)-Palladiumdichlorid infrage. Es kann auch ein Palladiumkomplex in der Reaktionsmischung erzeugt werden, wenn man ein Palladiumsalz und ein Komplexligand getrennt zur Reaktion zugibt.

Als Liganden kommen vorzugsweise Organophosphorverbindungen infrage. Beispielhaft seien genannt: Triphenylphosphin, tri-o-Tolylphosphin, 2,2'-Bis(diphenylphosphino)-1,1'-binaphthyl, Dicyclohexylphosphinebiphenyl, 1,4-Bis(diphenylphosphino)butan, Bisdiphenylphosphinoferrocen, Di(tert.-butylphosphino)biphenyl, Di(cyclohexylphosphino)biphenyl, 2-Dicyclohexylphosphino-2'-N,N-dimethylaminobiphenyl, tricyclohexylphosphine, tri-tert.-butylphosphine. Es kann aber auch auf Liganden verzichtet werden.

Das erfindungsgemäße Verfahren (g) wird ferner gegebenenfalls in Gegenwart eines weiteren Metallsalzes, wie Kupfersalzen, beispielsweise Kupfer-(I)-iodid durchgeführt.

Die Reaktionstemperaturen können bei der Durchführung des erfindungsgemäßen Verfahrens (g) in einem größeren Bereich variiert werden. Im allgemeinen arbeitet man bei Temperaturen von 20°C bis 180°C, vorzugsweise bei Temperaturen von 50°C bis 150°C.

Zur Durchführung des erfindungsgemäßen Verfahrens (g) zur Herstellung der Verbindungen der Formel (I) setzt man pro mol des Halogenoxathiincarboxamides der Formel (IV) im Allgemeinen 1 bis 5 mol, vorzugsweise 1 bis 2 mol an Alkin der Formel (IX) oder Alken der Formel (X) ein.

Als Verdünnungsmittel zur Durchführung des erfindungsgemäßen Verfahrens (h) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether.

Das erfindungsgemäße Verfahren (h) wird gegebenenfalls in Gegenwart eines geeigneten Säureakzeptors durchgeführt. Als solche kommen alle üblichen starken Basen infrage. Hierzu gehören vorzugsweise Erdalkalimetall- oder Alkalimetallhydride, -hydroxide, -amide, -alkoholate oder Alkalimetall-Kohlenwasserstoffverbindungen, wie beispielsweise Natriumhydrid, Natriumhydroxid, Kaliumhydroxid, Natriumamid, Litiumdiisopropylamid, Natrium-methylat, Natrium-ethylat; Kalium-tert.-butylat, Methylitium, Phenyllitium oder Butyllitium.

Die Reaktionstemperaturen können bei der Durchführung des erfindungsgemäßen Verfahrens (h) in einem größeren Bereich variiert werden. Im allgemeinen arbeitet man bei Temperaturen von -80°C bis 150°C, vorzugsweise bei Temperaturen von -30°C bis 80°C.

Zur Durchführung des erfindungsgemäßen Verfahrens (h) zur Herstellung der Verbindungen der Formel (I) setzt man pro mol des Ketons der Formel (XI) im Allgemeinen 1 bis 5 mol, vorzugsweise 1 bis 2 mol an Phosphorverbindung der Formel (XII) ein.

Als Verdünnungsmittel zur Durchführung des erfindungsgemäßen Verfahrens (i) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Düsopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (i) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (i) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (i) zur Herstellung der Verbindungen der Formel (I) setzt man pro Mol des Oxathiincarboxamids der Formel (Ib) im Allgemeinen 0,2 bis 5 Mol, vorzugsweise 0,5 bis 2 Mol an Halogenid der Formel (XIII) ein. Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obst- und Gemüseanbau, wie beispielsweise gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Altemaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthäline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychinolinsulfat; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomotph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamyein A; Vinclozolin; Zineb; Ziram; Zoxamide; (2S)-N-[2-[4-[[3-(4-Chlorphenyl)-2-propinyl]-oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3 pyridinyl)-cyclopropancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid, Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cyperinethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthtin; Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine, DDT, Deltamethin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (1R-isomer), Endosulfan; Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl, Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zur injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditiv ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Namatoden und Schnecken durch in den Pflanzen entstehende Toxine, insbesondere solche, die durch das genetische Material aus Bacillus Thuringiensis (z.B. durch die Gene CrylA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

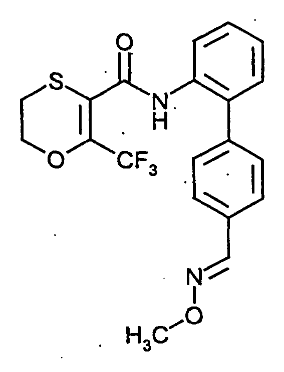

### Verfahren a)

Eine Mischung aus 6 ml Dichlormethan, 100 mg (0,467 mmol) 6-Trifluormethyl-2,3-dihydro-1,4-oxathiin-5-carbonsäure, 88 mg (0,389 mmol) 2'-Amino-biphenyl-4-carbaldehyd O-methyl-oxim, 100,6 mg (0,778 mmol) N,N-Diisopropylethylamin und 272 mg (0,584 mmol) Brom-tripyrrolidinophosphonium-hexafluorophosphat wird 2 Tage bei Raumtemperatur gerührt. Anschließend wird das Reaktionsgemisch mit 10 ml Wasser versetzt, die organische Phase abgetrennt und zuerst mit 10 ml gesättigter Ammoniumchloridlösung, danach mit 10 ml Wasser gewaschen, über Natriumsulfat getrocknet und eingedampft. Das Rohprodukt wird mittels präparativer HPLC (reversed phase Kieselgel (C₁₈), Laufmittel: Wasser/Acetonitril 34:66) gereinigt.
Man erhält: 35 mg (21 % der Theorie) 6-Trifluormethyl-2,3-dihydro-1,4-oxathiin-5-carbonsäure [4'-(methoxyimino-methyl)-biphenyl-2-yl]-amid mit dem logP (pH 2.3) = 3.51.

### Beispiel 2

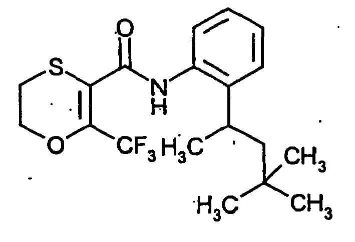

### Verfahren a)

300 mg (1.7 mmol) 6-Trifluormethyl-2,3-dihydro-1,4-oxathiin-5-carbonsäurechlorid und 220 mg (1.1 mmol) 2-(1,3,3-Trimethyl-butyl)-phenylamin werden in 5 ml Acetonitril 4 Tage bei Raumtemperatur im abgeschlossenen Gefäß gerührt. Anschließend wird das Reaktionsgemisch mit 10 ml Wasser versetzt, die organische Phase abgetrennt und zuerst mit 10 ml gesättigter Ammoniumchloridlösung, danach mit 10 ml Wasser gewaschen, über Natriumsulfat getrocknet und eingedampft. Das Rohprodukt wird mittels präparativer HPLC (reversed phase Kieselgel (C₁₈), Laufmittel: 2%ige Essigsäure/Acetonitril 25:75) gereinigt.
Man erhält: 200 mg (37 % der Theorie) 6-Trifluormethyl-2,3-dihydro-1,4-oxathiin-5-carbonsäure [2-(1,3,3-trimethyl-butyl)-phenyl]-amid mit dem logP (pH 2.3) = 4.18.

Analog Beispiel 1 und 2, sowie entsprechend den Angaben in der allgemeinen Beschreibung der erfindungsgemäßen Herstellverfahren (a) bis (h) wurden auch die in der nachstehenden Tabelle 1 genannten Verbindungen der Formel (I) erhalten:

**Tabelle 1**

| | | | | | | | | | | | |
|---|---|---|---|---|---|---|---|---|---|---|---|
| 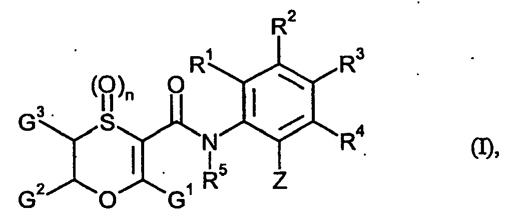 | | | | | | | | | | | |

| **Nr.** | **Z** | **R⁴** | **R³** | **R²** | **R¹** | **R⁵** | **n** | **G²** | **G³** | **G¹** | **logP *Fp*.** |
|---|---|---|---|---|---|---|---|---|---|---|---|
| 3 | 1,3-Dimethyl-butyl | H | H | H | H | H | 0 | H | CH₃ | CF₃ | 4,24 |
| 4 | 1,3,3-Trimethyl-butyl | H | H | H | H | H | 0 | H | CH₃ | CF₃ | 4,53 |
| 5 | 4-Fluor-3-methylphenyl | H | H | H | H | H | 0 | H | CH₃ | CF₃ | 3,98 |
| 6 | 3,4-Dichlorphenyl | H | H | H | H | H | 0 | H | CH₃ | CF₃ | 4,26 |
| 7 | 4-Chlor-3-trifluormethylphenyl | H | H | H | H | H | 0 | H | CH₃ | CF₃ | 4,30 |
| 8 | 4-Isopropoximinomethylphenyl | H | H | H | H | H | 0 | H | CH₃ | CF₃ | 4,57 |
| 9 | 4-Trifluormethylphenyl | H | H | H | H | H | 0 | H | CH₃ | CF₃ | 4,06 |
| 10 | Cycloheptyl | H | H | H | H | H | 0 | H | H | CF₃ | *137°C* |
| 11 | Cyclopentyl | H | H | H | H | H | 0 | H | H | CF₃ | *159°C* |
| 12 | -CH(CH₃)-CH₂-C(CH₃)₂- | | H | H | H | H | 0 | H | H | CF₃ | *165°C* |
| 13 | Cyclohexyl | H | F | H | H | H | 0 | H | H | CF₃ | *204-205°C* |
| 14 | Cyclohexyl | H | Cl | H | H | H | 0 | H | H | CF₃ | *169-171°C* |
| 15 | Bicyclo[2.2.1]hept-2-yl | H | H | H | H | H | 0 | H | H | CF₃ | *149°C* |
| 16 | Cyclooctyl | H | H | H | H | H | 0 | H | H | CF₃ | *102°C* |
| 17 | 3,4-Dichlorphenyl | H | F | H | H | H | 0 | H | H | CF₃ | 3,95 |
| 18 | 1,3-Dimethyl-butyl | H | H | H | H | H | 0 | H | H | CF₃ | 3,92 |
| 19 | 3,4-Dichlorphenyl | H | H | H | H | H | 0 | H | H | CF₃ | 3,91 |
| 20 | 4-Trifluormethylphenyl | H | H | H | H | H | 0 | H | H | CF₃ | 3,73 |
| 21 | 4-Isopropoximinomethyphenyl | H | H | H | H | H | 0 | H | H | CF₃ | 4,22 |
| 22 | 4-Propoxyiminomethyl | H | H | H | H | H | 0 | H | H | CF₃ | 4,25 |
| 23 | 4-Chlor-3-fluorphenyl | H | H | H | H | H | 0 | H | H | CF₃ | 3,62 |
| 24 | 3-Chlor-4-fluorphenyl | H | H | H | H | H | 0 | H | H | CF₃ | 3,60 |
| 25 | 3-Fluor-4-propoxyiminomethyl-phenyl | H | H | H | H | H | 0 | H | H | CF₃ | 4,42 |
| 26 | 4-Chlor-2-methylphenyl | H | H | H | H | H | 0 | H | H | CF₃ | 4,00 |
| 27 | 4-Bromphenyl | H | H | H | H | H | 0 | H | H | CF₃ | 3,70 |
| 28 | Cyclohexyl | H | H | H | H | H | 0 | H | H | CF₃ | 3,69 |
| 29 | 3-Fluor-4-trifluormethylphenyl | H | H | H | H | H | 0 | H | H | CF₃ | 3,75 *114-116°C* |
| 30 | 4-Chlor-3-trifluormethylphenyl | H | H | H | H | H | 0 | H H | H | CF₃ | 4,03 |
| 31 | (2-Cyclohexyl)-1-methyl-ethyl | H | H | H | H | H | 0 | H | H | CF₃ | 4,71 |
| 32 | 3,5-Difluorphenyl | H | H | H | H | H | 0 | H | H | CF₃ | 3,35 *167°C* |
| 33 | 4-Iodphenyl | H | H | H | H | H | 0 | H | H | CF₃ | 3,85 |
| 34 | 1-Methyl-3-butenyl | H | H | H | H | H | 0 | H | H | CF₃ | 3,33 |
| 35 | 4-Fluor-3-methylphenyl | H | H | H | H | H | 0 | H | H | CF₃ | 3,65 |
| 36 | 3-Fluor-4-methylphenyl | H | H | H | H | H | 0 | H | H | CF₃ | 3,62 |
| 37 | (2-Cyclopentyl)-1-methyl-ethyl | H | H | H | H | H | 0 | H | H | CF₃ | 4,38 |
| 38 | 3-Chlor-4-methylphenyl | H | H | H | H | H | 0 | H | H | CF₃ | 3,89 |
| 39 | 1-Methyl-butyl | H | H | H | H | H | 0 | H | H | CF₃ | 3,63 |
| 40 | 4-(1-(3-Propinyloxyimino)-ethyl)-phenyl | H | H | H | H | H | 0 | H | H | CF₃ | 3,76 *125-127°C* |
| 41 | 4-(1-Amino-1-methoxyimino-methyl)-phenyl | H | H | H | H | H | 0 | H | H | CF₃ | 1,96 |
| 42 | 1-Methyl-nonyl | H | H | H | H | H | 0 | H | H | CF₃ | 5,48 |
| 43 | 4-Brom-2-fluorphenyl | H | H | H | H | H | 0 | H | H | CF₃ | 3,57 |
| 44 | 3-Chlor-5-fluor-phenyl | H | H | H | H | H | 0 | H | H | CF₃ | 3,60 |
| 45 | 3-Chlor-4-(1-methoxyimino-ethyl)-phenyl | H | H | H | H | H | 0 | H | H | CF₃ | 3,45 |
| 46 | 3-Fluor-4-(1-methoxyimino-ethyl)-phenyl | H | H | H | H | H | 0 | H | H | CF₃ | 3,69 |
| 47 | Bicyclo[2.2.1]hept-2-yl | H | H | H | H | H | 0 | H | H | CF₃ | 4,32 |
| 48 | 1-Methyl-hexyl | H | H | H | H | H | 0 | H | H | CF₃ | 4,34 |
| 49 | 1-Cyclohexylethyl | H | H | H | H | H | 0 | H | H | CF₃ | 4,31 |
| 50 | 1,3-Dimethyl-butyl | H | F | H | H | H | 0 | H | H | CF₃ | 3,94 |
| 51 | 1-Ethyl-propyl | H | H | H | H | H | 0 | H | H | CF₃ | 3,62 |
| 52 | 1-Methyl-butyl | H | F | H | H | H | 0 | H | H | CF₃ | 3,67 |
| 53 | 3-Chlor-4-trifluormethylphenyl | H | H | H | H | H | 0 | H | H | CF₃ | 3,98 *100*- *102°C* |
| 54 | 1,3-Dimethyl-pentyl | H | H | H | H | H | 0 | H | H | CF₃ | 4,25 |
| 55 | 2-(2,2-Dichlorcyclopropyl)-1-methyl-ethyl | H | H | H | H | H | 0 | H | H | CF₃ | 3,92 |
| 56 | 4-(1-Methoxyiminopropyl)-phenyl | H | H | H | H | H | 0 | H | H | CF₃ | 4,08 |
| 57 | 4-Brom-3-fluor-phenyl | H | H | H | H | H | 0 | H | H | CF₃ | 3,69 |
| 58 | 1,3,3-Trimethyl-pentyl | H | H | H | H | H | 0 | H | H | CF₃ | 4,48 |
| 59 | Bicyclo[2.2.1]hept-2-yl | H | H | H | H | H | 0 | CH₃ | H | CF₃ | 5,36 |
| 60 | -CH(CH₃)-CH₂-C(CH₃)₂- | | H | H | H | H | 0 | H | H | CF₃ | 4,18 |
| 61 | 4-Trifluormethylphenyl | H | H | H | H | H | 0 | CH₃ | H | CF₃ | 4,08 |
| 62 | 4-Methoxyiminomethylphenyl | H | H | H | H | H | 0 | CH₃ | H | CF₃ | 3,81 |
| 63 | 1,3-Dimethyl-butyl | H | H | H | H | H | 0 | CH₃ | H | CF₃ | 4,27 |
| 64 | 4-Isopropoximinomethylphenyl | H | H | H | H | H | 0 | CH₃ | H | CF₃ | 4,56 |
| 65 | 3,4-Dichlorphenyl | H | H | H | H | H | 0 | CH₃ | H | CF₃ | 4,27 |
| 66 | 1,3,3-Trimethyl-butyl | H | H | H | H | H | 0 | CH₃ | H | CF₃ | 4,51 |
| 67 | 3,4-Dichlorphenyl | H | F | H | H | H | 0 | CH₃ | H | CF₃ | 4,32 |
| 68 | 3-Fluor-4-propoxyiminomethyl-phenyl | H | H | H | H | H | 0 | CH₃ | H | CF₃ | 4,77 |
| 69 | 3-Fluor-4-methylphenyl | H | H | H | H | H | 0 | CH₃ | H | CF₃ | 3,99 |
| 70 | 4-Fluor-3-methylphenyl | H | H | H | H | H | 0 | CH₃ | H | CF₃ | 3,99 |
| 71 | 3-Fluor-4-trifluormethylphenyl | H | H | H | H | H | 0 | CH₃ | H | CF₃ | 4,08 |
| 72 | 2-Chlor-4-methoxyiminophenyl | H | H | H | H | H | 0 | H | H | CF₃ | 3,81 |
| 73 | 3,5-Difluorphenyl | H | F | H | H | H | 0 | H | H | CF₃ | 3,49 |
| 74 | 3,5-Difluorphenyl | H | H | H | F | H | 0 | H | H | CF₃ | 3,20 |
| 75 | 3,5-Difluorphenyl | H | H | F | H | H | 0 | H | H | CF₃ | 3,70 |
| 76 | n-Hexyl | H | H | H | H | H | 0 | H | H | CF₃ | 4,25 |
| 77 | 1-Ethyl-butyl | H | H | H | H | H | 0 | H | H | CF₃ | 4,07 |
| 78 | 4-Cyanophenyl | H | H | H | H | H | 0 | H | H | CF₃ | 2,87 |
| 79 | 2,4-Dichlorphenyl | H | H | H | H | H | 0 | H | H | CF₃ | 3,82 |
| 80 | 3,4-Dichlorphenyl | H | H | H | H | H | 0 | H | H | Cyclopropyl | 3,53 |
| 81 | 1,3,3-Trimethyl-butyl | H | H | H | H | H | 0 | H | H | CHF₂ | 4,29 |
| 82 | 1,3-Dimethyl-butyl | H | H | H | H | H | 0 | H | H | CHF₂ | 4,03 |
| 83 | -CH(CH₃)-CH₂-C(CH₃)₂- | | H | H | H | H | 0 | H | H | CHF₂ | 4,00 |
| 84 | 3,4-Dichlorphenyl | H | H | H | H | H | 0 | H | H | CHF₂ | 3,92 |
| 85 | 3-Fluor-4-propoxyiminomethyl-phenyl | H | H | H | H | H | 0 | H | H | CHF₂ | 4,52 |
| 86 | 4-Methoxyiminomethylphenyl | H | H | H | H | H | 0 | H | H | CHF₂ | |
| 87 | 3-Methyl-1-propyl-butyl | H | H | H | H | H | 0 | H | H | CF₃ | 4,65 |
| 88 | 3-Methyl-1-ethyl-butyl | H | H | H | H | H | 0 | H | H | CF₃ | 4,30 |
| 89 | 1,3-Dimethyl-1-butenyl | H | H | H | H | H | 0 | H | H | CF₃ | 4,32 |
| 90 | 1,3-Dimethyl-butyl | H | H | H | H | H | 2 | H | H | CF₃ | 3,53 |
| 91 | 4-Bromphenyl | H | H | H | H | H | 2 | H | H | CF₃ | 3,35 |
| 92 | 4-Chlor-2-methylphenyl | H | H | H | H | H | 2 | H | H | CF₃ | 3,66 |
| 93 | 3,4-Dichlorphenyl | H | H | H | H | H | 2 | H | H | CF₃ | 3,57 |
| 94 | 1,2-Dimethyl-butyl | H | H | H | H | H | 0 | H | H | CF₃ | 3,88 |
| 95 | 4-Chlor-3-fluorphenyl | H | H | H | H | H | 2 | H | H | CF₃ | 3,28 |
| 96 | (2-Cyclopropyl)-1-methyl-ethyl | H | H | H | H | H | 0 | H | H | CF₃ | 3,72 |
| 97 | 3,3-Dimethyl-1-butinyl | H | H | H | H | H | 0 | H | H | CF₃ | 4,42 |
| 98 | 4-Brom-3-methylphenyl | H | H | H | H | H | 0 | H | H | CF₃ | 4,06 |
| 99 | 1,3-Dimethyl-butyl | H | H | H | H | -COCH₃ | 0 | H | H | CF₃ | 4,56 |
| 100 | 4-(2,2,2-Trifluor-N-methoxyethanimidoyl)phenyl | H | H | H | H | H | 0 | H | H | CF₃ | 2.53 |
| 101 | 2,2-Dichlor-1-methylcyclopropyl | H | H | H | H | H | 0 | H | H | CF₃ | 3.66 |
| 102 | 3-Methylbutyl | H | H | H | H | H | 0 | H | H | CF₃ | 3.72 |
| 103 | 3,3-Dimethylbutyl | H | H | H | H | H | 0 | H | H | CF₃ | 3.98 |

### Herstellung der Vorstufen der Formel (II)

### Beispiel (II-1)

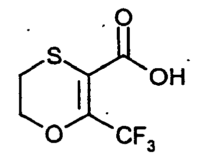

### Erste Stufe (II-1a):

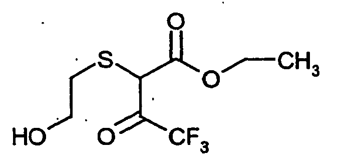

Zu einer Lösung von 20,0 g (0,092 mol) Ethyl-2-chlor-3-keto-4,4,4-trifluorbutyrat in 100 ml Toluol tropft man bei ca. 5°C 10,17 g (0,09 Mol) Triethylamin. Danach wird innerhalb von 1 Stunde eine Lösung von 7,0 g (0,09 Mol) 2-Mercaptoethanol in 5 ml Toluol zugetropft und 2 Stunden bei ca. 5°C nachgerührt. Die Suspension wird abfiltriert und mit wenig Toluol nachgewaschen. Das Filtrat wird zuerst mit 50 ml 1N Salzsäure, dann zweimal mit 50 ml gesättigter Natriumhydrogencarbonatlösung, zuletzt mit 50 ml Wasser gewaschen, über Magnesiumsulfat getrocknet und eingedampft.
Man erhält: 18,8 g (62 % der Theorie) 4,4,4-Trifluor-2-(2-hydroxy-ethylsulfanyl)-3-oxo-buttersäureethylester (vgl. Heterocycles, 1998, 48, 2253-2262).

### Zweite Stufe (II-1b):

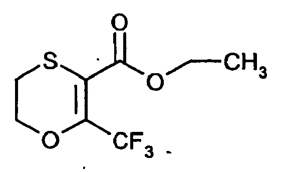

Zu einer Lösung von 18,7g (0,071 mol) 4,4,4-Trifluor-2-(2-hydroxy-ethylsulfanyl)-3-oxo-buttersäureethylester (II-1a) in 60 ml Toluol gibt man zuerst 6,25g (0,079Mol) Pyridin und tropft danach innerhalb von 30 Minuten bei 20°C 4 g (0,079Mol) Thionylchlorid zu. Aufgrund der exothermen Reaktion muss die Temperatur der Reaktionsmischung mit Eiswasser gekühlt werden. Nach Ende der heftigen Reaktion wird noch 5 Stunden bei Raumtemperatur gerührt. Die Mischung wird filtriert und der Rückstand mit 10 ml Toluol gewaschen. Zum Filtrat wird bei ca. 10°C innerhalb von 1 Stunde 10,9 g (0,108 Mol) Triethylamin getropft und für 16 Stunden bei Raumtemperatur gerührt. Das Reaktionsgemisch wird zuerst zweimal mit 50 ml 1N Salzsäure, dann zweimal mit 50 ml gesättigter Natriumhydrogencarbonatlösung, zuletzt mit 50 ml Wasser gewaschen, über Magnesiumsulfat getrocknet und eingedampft. Der Rückstand wird mit Petrolether/Essigester (10:1 bis 1:1) an Kieselgel chromatografiert.
Man erhält 12,6 g (59,4 % der Theorie) 6-Trifluormethyl-2,3-dihydro-1,4-oxathiin-5-carbonsäureethylester (vgl. Heterocycles 1998, 48, 2253-2262).

### Dritte Stufe:

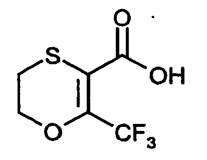

12,6 g (0,05 mol) 6-Trifluormethyl-2,3-dihydro-1,4-oxathiin-5-carbonsäureethylester (II-1b) und 3,14 g (0,08 mol) Natriumhydroxid werden in 50 ml Wasser 1 Stunde unter Rückfluss gekocht. Das Reaktionsgemisch wird abgekühlt und zweimal mit Dichlormethan extrahiert. Die wässrige Phase wird mit 6 N Salzsäure auf pH 2 angesäuert, dann 5 mal mit Diethylether extrahiert. Die vereinigten organischen Extrakte werden über Natriumsulfat getrocknet und eingedampft.
Man erhält: 9,2 g (82 % der Theorie) 6-Trifluormethyl-2,3-dihydro-1,4-oxathiin-5-carbonsäure.

Analog Beispiel (II-1) wurden auch die in nachstehend genannten Verbindungen der Formel (II) erhalten:

### Beispiel (II-2)

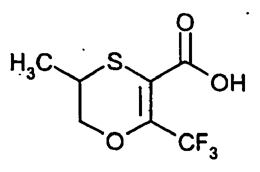

HPLC: logP = 1,69

### Beispiel (II-3)

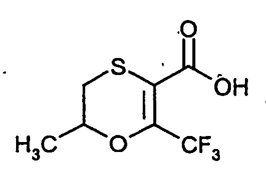

HPLC: logP = 1,72

### Beispiel (II-4)

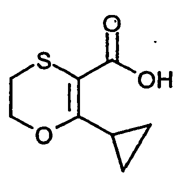

### Beispiel (II-5)

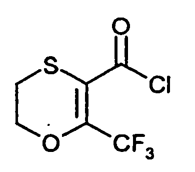

4.5 g (21 mmol) 6-Trifluormethyl-2,3-dihydro-1,4-oxathiin-5-carbonsäure (II-1) und 21 g Thionylchlorid werden in 10 ml Toluol 4 Stunden bei 80°C gerührt. Das Ende der Reaktion wird festgestellt, indem aus der Reaktionslösung regelmäßig Proben entnommen und mit Methanol versetzt werden, wovon dann Dünnschichtchromatogramme angefertigt werden.

Nach Ende der Reaktion werden flüchtige Bestandteile abdestilliert. Der Rückstand wird dreimal mit jeweils 20 ml Dichlormethan verrührt und wieder eingedampft.

Man erhält 4,5 g (80 % der Theorie) 6-Trifluormethyl-2,3-dihydro-1,4-oxathiin-5-carbonsäurechlorid. Das Produkt wurde charakterisiert, indem eine Probe davon 2 Stunden mit Methanol gekocht wurde und der so erhaltene 6-Trifluormethyl-2,3-dihydro-1,4-oxathiin-5-carbonsäuremethylester mittels GC/MS nachgewiesen wurde.

### Herstellung der Vorstufen der Formel (III)

### Beispiel (III-1)

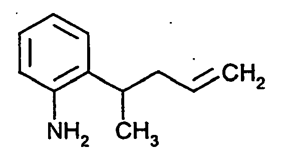

### Erste Stufe (III-1a)

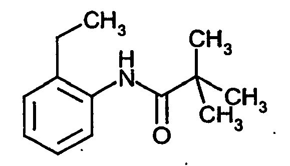

Zu einer Lösung von 15 g (124 mmol) 2-Ethylanilin, 25 g (248 mMol) Triethylamin in 150 ml Dichlormethan tropft man bei 0° C das 15 g (124 mmol) Pivalinsäurechlorid und rührt 2 Stunden bei 0°C und anschließend ohne weitere Kühlung für 16 Stunden. Das Gemisch wird mit Dichlormethan verdünnt, zuerst mit Wasser dann mit Ammoniumchlorid-Lösung gewaschen, die organische Phase abgetrennt, über Natriumsulfat getrocknet und eingeengt.

Man erhält 25 g (98 % der Theorie) *N*-(2-Ethyl-phenyl)-2,2-dimethyl-propionamid.

### Zweite Stufe (III-1b)

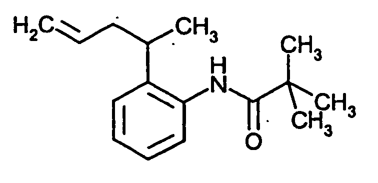

Zu einer Lösung aus 5,6 g (0,0275 Mol) *N*-(2-Ethyl-phenyl)-2,2-dimethyl-propionamid (IIIla) in 150 ml n-Pentan/Methyl-t-butylether (1:1) gibt man unter Argon bei -25 °C zuerst 3,7 g (0,03 Mol) Diazabicyclo[2.2.2]octan (DABCO) dann 44,5 ml (0,058 mol) sec-Butyllithium-Lösung (1,3 molar in Hexan) und rührt weitere 2 Stunden bei der gleichen Temperatur. Nun wird das Gemisch auf -78 °C gekühlt, 3,33 g (0,0275 Mol) Allylbromid zugetropft und 60 Minuten bei -60 bis -78 °C gerührt. Reste von sec-Butyllithium werden durch Zugabe von 30 ml Methanol zersetzt. Das Gemisch wird auf Raumtemperatur erwärmt, mit 50 ml 5%iger Phosphorsäure geschüttelt. Die organische Phase wird abgetrennt, über Kieselgel filtriert und mittels präparativer HPLC (reversed phase Kieselgel (C₁₈), Laufmittel: Wasser/Acetonitril 38:62) gereinigt.

Man erhält 1,8 g (26 % der Theorie) 2,2-Dimethyl-*N*-[2-(1-methyl-but-3-enyl)-phenyl]-propionamid.

### Dritte Stufe (III-1)

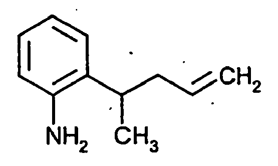

1,5g (6,1 mmol) 2,2-Dimethyl-*N*-[2-(1-methyl-but-3-enyl)-phenyl]-propionamid (III-1b) und 1,2 g (12,2 mmol) konzentrierter Salzsäure werden in 24,5 ml Dioxan 12 Stunden bei 80 °C gerührt. Das Reaktionsgemisch wird mit 10 ml Wasser versetzt und mit verdünnter Natronlauge alkalisch gestellt und 5 mal mit Essigsäureethylester extrahiert. Die vereinigten, organischen Extrakte werden über Natriumsulfat getrocknet und eingedampft, über Kieselgel filtriert (Essigester/Methanol 5:1) und mittels präparativer HPLC (reversed phase Kieselgel (C₁₈), Laufmittel: 2%ige Essigsäure/Acetonitril 70:30) gereinigt
Man erhält 0,3 g (30 % der Theorie) 2-(1-Methyl-but-3-enyl)-phenylamin.

Analog Beispiel (III-1) wurden auch die in nachstehend genannten Verbindungen der Formel (III) erhalten:

### Beispiel (III-2)

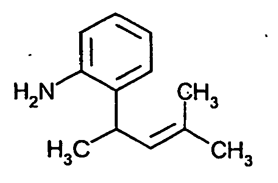

HPLC: logP = 1,19

### Beispiel (III-3)

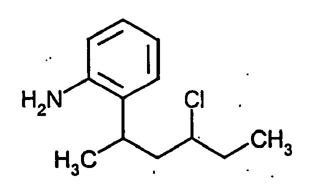

HPLC: logP = 2,3

2-(3-Chlor-1-methylpentyl)phenylamin (III-3) wird erhalten, indem man zunächst *N*-[2-(2-Cyclopropyl-isopropyl)phenyl]-2,2-dimethylpropanamid analog den Beispielen (III-1a) und (III-1b) herstellt. In der dritten Stufe wird mit Salzsäure umgesetzt, wobei neben 2-(2-Cyclopropyl-isopropyl)phenylamin auch die Verbindung (III-3) erhalten wird.

### Beispiel (III-4)

### Erste Stufe (III-4a)

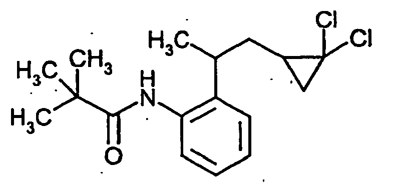

Zu einer Lösung von 2,2-Dimethyl-N-[2-(1-methyl-but-3-enyl)-phenyl]-propionamid (III-1b) 900 mg (3,7 mmol) in 5 ml Chloroform/Wasser (1:1) gibt man nacheinander bei Raumtemperatur 100 mg Benzyltriethylammoniumchlorid und 1 ml 50%ige Natronlauge und rührt 5 Tage. Das Reaktionsgemisch wird mit 20 ml Chloroform weiter verdünnt, mit 50 ml Natriumchlorid-Lösung gewaschen, die organische Phase abgetrennt, über Natriumsulfat getrocknet und eingeengt.

Man erhält 800 mg (37 % der Theorie) *N*-{2-[2-(2,2-Dichlorcymopropyl)-1-methylethyl]-phenyl}-2,2-dimethyl-propionamid mit dem logP (pH 2,3) = 3,77.

### Zweite Stufe (III-4b)

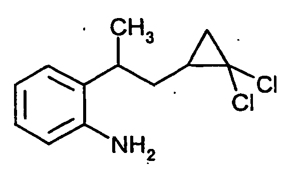

800 mg (1,34 mmol) N-{2-[2-(2,2-Dichlorcyclopropyl)-1-methylethyl]-phenyl}-2,2-dimethyl-propionamid (III-4a) werden in 25 ml Dioxan gelöst und mit 0,1 ml konzentrierter Salzsäure versetzt, 72 Stunden bei Raumtemperatur und 24 Stunden bei 80°C gerührt. Das Gemisch wird mit Eiswasser verdünnt, mit konzentrierter Natronlauge alkalisch gestellt und mit 50 ml Essigsäureethylester extrahiert. Die organische Phase wird über Natriumsulfat getrocknet und bei vermindertem Druck eingeengt. Der Rückstand wird über Kieselgel mit Methanol filtriert und mittels präparativer HPLC (reversed phase Kieselgel (C₁₈), Laufmittel: 2%ige Posphorsäure/Àcetonitril 52:48) gereinigt.

Man erhält 0,8 g (60 % der Theorie) 2-[2-(2,2-Dichlorcyclopropyl)-1-methylethyl]-phenylamin mit dem logP (pH 2,3) = 2,52.

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

Eluenten für die Bestimmung im sauren Bereich (pH 2,3): 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Venturia - Test (Apfel) / protektiv

| | | |
|---|---|---|
| Lösungsmittel : | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

| Tabelle A: | | | |
|---|---|---|---|
| **Venturia - Test (Apfel) / protektiv** | | | |
| Bsp. | Wirkstoff | Aufwandmenge an Wirkstoff in g/ha | % Wirkungsgrad |
| 12 | 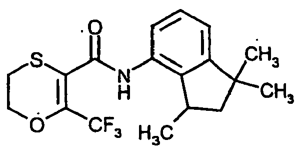 | 100 | 100 |
| 17 | 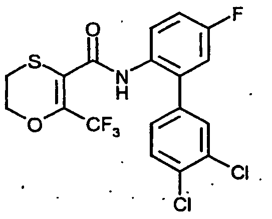 | 100 | 100 |
| 20 | 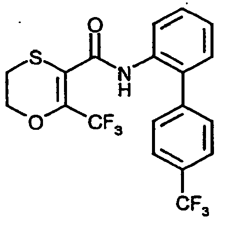 | 100 | 88 |
| 18 | 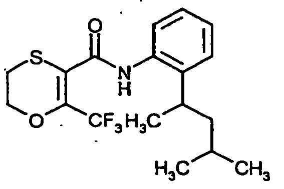 | 100 | 100 |
| 19 | 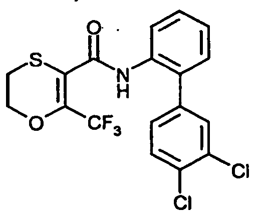 | 100 | 100 |
| 21 | 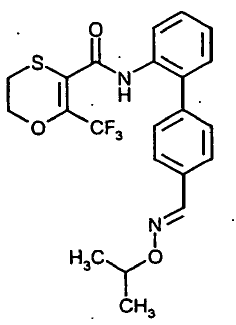 | 100 | 100 |
| 22 | 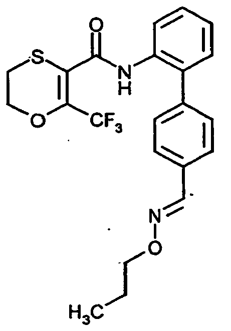 | 100 | 100 |
| 2 | 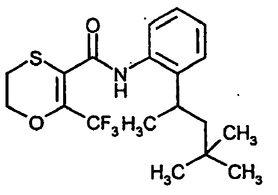 | 100 | 98 |

### Beispiel B

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

| Tabelle B: | | | |
|---|---|---|---|
| **Botrytis - Test (Bohne) / protektiv** | | | |
| Bsp. | Wirkstoff | Aufwandmenge an Wirkstoff in g/ha | % Wirkungsgrad |
| 12 | 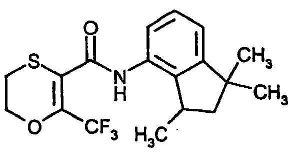 | 500 | 97 |
| 17 | 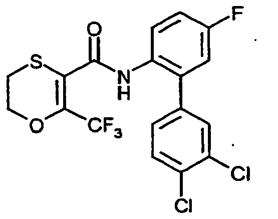 | 500 | 96 |
| 20 | 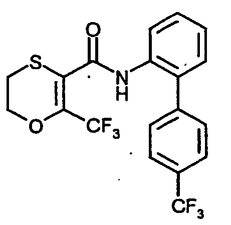 | 500 | 100 |
| 18 | 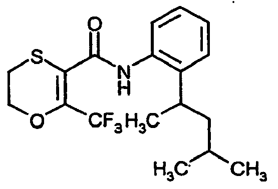 | 500 | 100 |
| 19 | 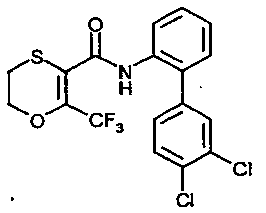 | 500 | 100 |
| 21 | 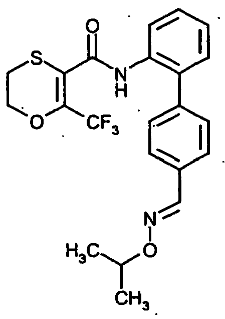 | 500 | 100 |
| 22 | 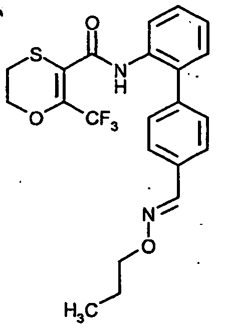 | 500 | 100 |
| 2 | 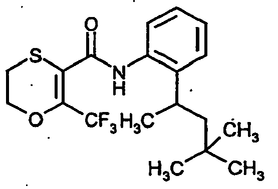 | 500 | 100 |

### Beispiel C

### Alternaria-Test (Tomate) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Alternaria solani inokuliert und stehen dann 24 h bei 100 % relativer Feuchte und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmenge und Versuchsergebnisse gehen aus der folgenden Tabelle hervor.

| Tabelle C: | | | |
|---|---|---|---|
| **Alternaria-Test (Tomate) / protektiv** | | | |
| Bsp. | Wirkstoff | Aufwandmenge an Wirkstoff in g/ha | % Wirkungsgrad |
| 26 | 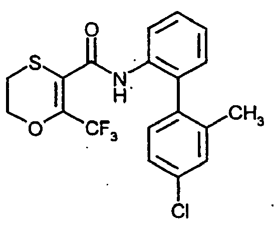 | 750 | 100 |
| 2 | 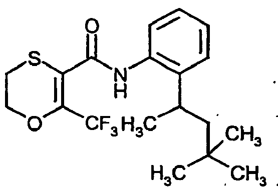 | 750 | 100 |
| 19 | 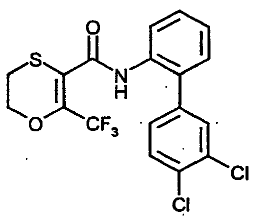 | 750 | 100 |
| 21 | 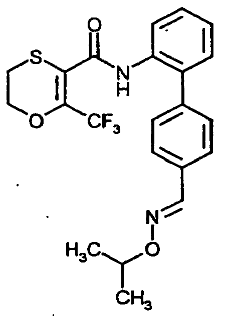 | 750 | 100 |

### Beispiel D

### Puccinia-Test (Weizen) / protektiv

| | |
|---|---|
| Lösungsmittel: | N,N-Dimethylacetamid |
| Emulgator: | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen, Lösungmittelmengen, Emulgatormengen und Versuchsergebnisse gehen aus der folgenden Tabelle hervor.

| Tabelle D: | | | | |
|---|---|---|---|---|
| **Puccinia-Test (Weizen) / protektiv** | | | | |
| Bsp. | Wirkstoff | Aufwandmenge an Wirkstoff in g/ha | % Wirkungsgrad | Gewichtsteile **Lösungsmittel/** *Emulgator* |
| 18 | 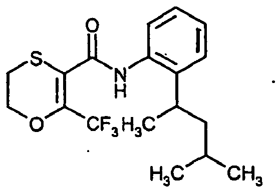 | 500 | 100 | **25**/*0.6* |
| 17 | 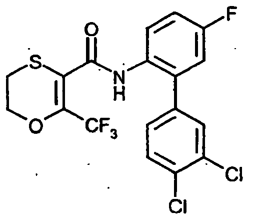 | 500 | 86 | **25**/*0.6* |
| 35 | 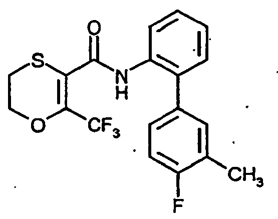 | 500 | 88 | **25**/*0.6* |
| 19 | 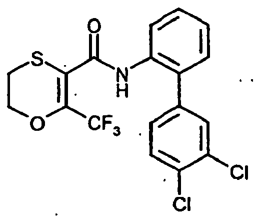 | 500 | 100 | **25**/*0.6* |
| 2 | 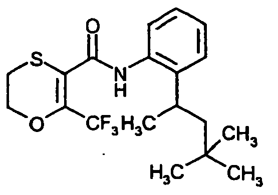 | 500 | 100 | **25**/*0.6* |
| 24 | 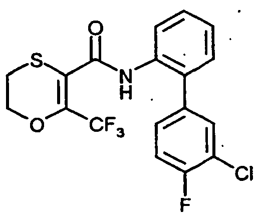 | 500 | 100 | **25**/*0.6* |
| 38 | 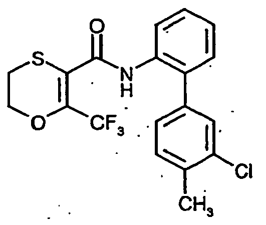 | 500 | 88 | **25**/*0.6* |
| 37 | 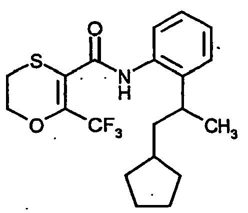 | 500 | 93 | **25**/*0.6* |
| 36 | 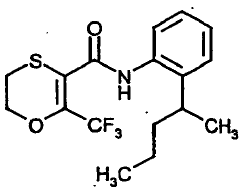 | 500 | 100 | **25**/*0.6* |
| 56 | 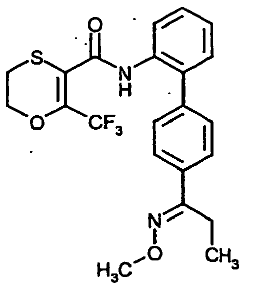 | 500 | 88 | **25**/*0.6* |
| 57 | 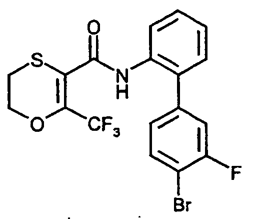 | 500 | 88 | **25**/*0.6* |
| 54 | 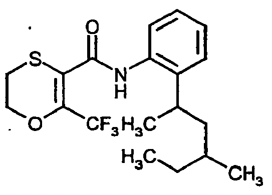 | 500 | 100 | **50**/*1.0* |
| 29 | 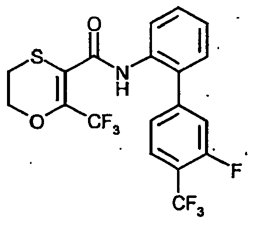 | 500 | 94 | **50**/*1.0* |
| 53 | 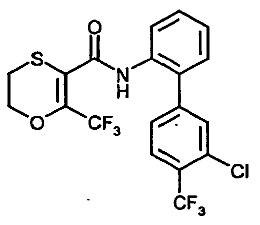 | 500 | 100 | **50**/*1.0* |
| 79 | 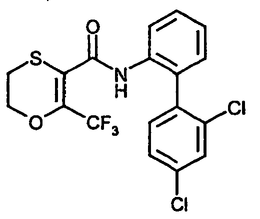 | 500 | 86 | **50**/*1.0* |
| 23 | 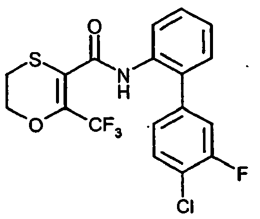 | 500 | 89 | **50**/*1.0* |
| 27 | 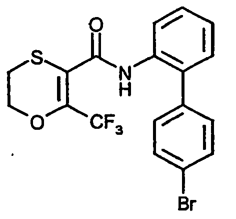 | 500 | 88 | **50**/*1.0* |
| 82 | 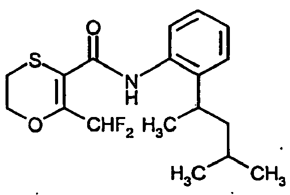 | 500 | 100 | **50**/*1.0* |

## Patentansprüche

1. Oxathiincarboxamide der Formel (I) 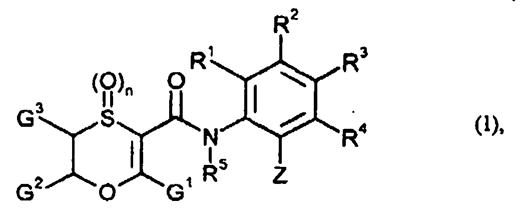 in welcher
G¹ für Halogen, Trifluormethyl, Difluormethyl oder Cyclopropyl steht,
G² und G³ unabhängig voneinander für Wasserstoff oder Methyl stehen,
n für 0, 1 oder 2 steht,
R¹, R², R³ und R⁴ unabhängig voneinander für Wasserstoff, Fluor, Chlor, Methyl, iso-Propyl oder Methylthio stehen,
R⁵ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatome, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁶, -CONR⁷R⁸ oder -CH₂NR⁹R¹⁰ steht,
R⁶ für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen; -COR" steht,
R⁷ und R⁸ unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R⁷ und R⁸ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R⁹ und R¹⁰ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R⁹ und R¹⁰ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R¹¹ für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
R¹² für Wasserstoff oder C₁-C₆-Alkyl steht,
Z für Z¹, Z², Z³ oder Z⁴ steht, worin
Z¹ für einfach bis fünffach, gleich oder verschieden substituiertes Phenyl steht,
Z² für gegebenenfalls einfach oder mehrfach, gleich oder verschieden substituiertes Cycloalkyl oder Bicycloalkyl steht,
Z³ für unsubstituiertes C₄-C₂₀-Alkyl oder für einfach oder mehrfach, gleich oder verschieden durch Halogen und/oder C₃-C₆-Cycloalkyl substituiertes C₁-C₂₀-Alkyl steht, wobei der Cycloalkylteil seinerseits gegebenenfalls einfach oder mehrfach, gleich oder verschieden durch Halogen und/oder C₁-C₄-Alkyl substituiert sein kann,
Z⁴ für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann,
oder
R¹, R² und R³ unabhängig voneinander für Wasserstoff oder Fluor stehen und
Z und R⁴ gemeinsam mit den Kohlenstoffatomen, an die sie gebunden sind, einen gegebenenfalls substituierten 5- oder 6-gliedrigen carbocyclischen oder heterocyclischen Ring bilden.

2. Qxathiincarboxamide der Formel (I) gemäß Anspruch 1, in welcher
G¹ für Fluor, Chlor, Brom, Iod, Trifluormethyl, Difluormethyl oder Cyclopropyl,
G² und G³ unabhängig voneinander für Wasserstoff oder Methyl stehen,
n für 0 oder 2 steht.

3. Oxathiincarboxamide der Formel (I) gemäß Anspruch 1, in welcher
R⁵ für Wasserstoff steht.

4. Oxathiincarboxamide der Formel (I) gemäß Anspruch 1, in welcher
R¹ für Wasserstoff, Fluor, Chlor oder Methyl steht,
R² für Wasserstoff, Fluor, Chlor, iso-Propyl oder Methylthio steht,
R³ für Wasserstoff, Fluor, Chlor oder Methyl steht,
R⁴ für Wasserstoff, Fluor, Chlor oder Methyl steht.

5. Oxathiincarboxamide der Formel (I) gemäß Anspruch 1, in welcher^
Z für Z¹ steht,
Z¹ für einfach bis fünffach, gleich oder verschieden substituiertes Phenyl, wobei die Substituenten aus der Liste W¹ ausgewählt sind,
W¹ für Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bits 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy mit 1 bis 6 Kohlenstoffatomen in den jeweiligen Kohlenwasserstoffketten, Alkenylcarbonyl oder Alkinylcarbonyl, mit 2 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 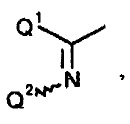 worin
Q¹ für Wasserstoff, Hydroxy oder Alkyl mit 1 bis 4 Kohlenstoffatomen, Halogenalkyl mit 1 bis 4 Kohlenstoffatomen und 1 bis 9 Fluor-, Chlor- und/oder Bromatomen oder Cycloalkyl mit 1 bis 6 Kohlenstoffatomen steht und
Q² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen, steht.

6. Oxathiincarboxamide der Formel (I) gemäß Anspruch 1, in welcher
Z für Z² steht,
Z² für jeweils gegebenenfalls einfach bis vierfach, gleich oder verschieden durch Halogen und/oder C₁-C₄-Alkyl substituiertes Cycloalkyl oder Bicycloalkyl mit jeweils 3 bis 10 Kohlenstoffatomen steht.

7. Oxathiincarboxamide der Formel (I) gemäß Anspruch 1, in welcher
Z für Z³ steht,
Z³ für unsubstituiertes C₄-C₂₀ oder für einfach oder mehrfach, gleich oder verschieden durch Fluor, Chlor, Brom, Iod und/oder C₃-C₆-Cycloalkyl substituiertes C₁-C₂₀-Alkyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann, steht.

8. Oxathiincarboxamide der Formel (I) gemäß Anspruch 1, in welcher
Z für Z⁴ steht,
Z⁴ für jeweils gegebenenfalls einfach oder mehrfach, gleich oder verschieden durch Fluor, Chlor, Brom, Iod und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann, steht.

9. Verfahren zum Herstellen der Oxathiincarboxamide der Formel (I) gemäß Anspruch 1, **dadurch gekennzeichnet, dass** man
a) Qxathiincarbonsäurederivate der Formel (II) 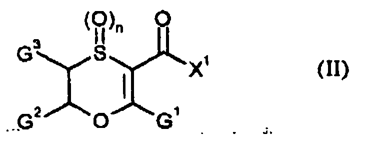 in welcher
G¹, G², G³ und n die in Anspruch 1 angegebenen Bedeutungen haben,
X¹ für Halogen oder Hydroxy steht,
mit Anilin-Derivaten der Formel (III) 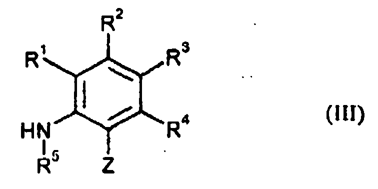 in welcher
R¹, R², R³, R⁴, R⁵ und Z die in Anspruch 1 angegebenen Bedeutungen haben,
gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder
b) Halogenoxathiincarboxamide der Formel (IV) 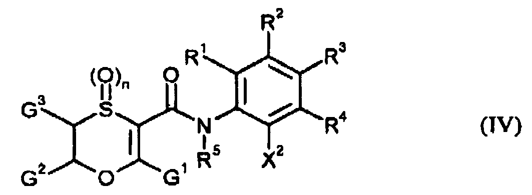 in welche
G¹, G², G³, n, R¹, R², R³, R⁴ und R⁵ die in Anspruch 1 angegebenen Bedeutungen haben,
X² für Brom oder Iod steht,
mit Boronsäure-Derivaten der Formel (V) 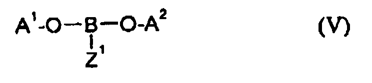 in welcher
Z¹ die in Anspruch 1 angegebenen Bedeutungen hat und
A¹ und A² jeweils für Wasserstoff oder zusammen für Tetramethylethylen stehen,
in Gegenwart eines Katalysators, gegebenenfalls in Gegenwart eines Säurebindemittels und gegebenenfalls in Gegenwart eines Verdünnungsmittels umsetzt, oder
c) Oxathiincarboxamid-Boronsäure-Derivate der Formel (VI) 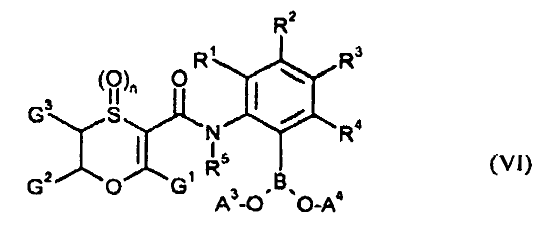 in welcher
G¹, G², G³, n, R¹, R², R³, R⁴ und R⁵ die in Anspruch 1 angegebenen Bedeutungen haben,
A³ und A⁴ jeweils für Wasserstoff oder zusammen für Tetramethylethylen stehen,
mit Phenyl-Derivaten der Formel (VII)
X³-Z¹ (VII)
in welcher
Z¹ die in Anspruch angegebenen Bedeutungen hat und
X³ für Chlor, Brom, Iod oder Trifluormethylsulfonat steht,
in Gegenwart eines Katalysators, gegebenenfalls in Gegenwart eines Säurebindemittels und gegebenenfalls in Gegenwart eines Verdünnungsmittels umsetzt, oder
d) Halogenoxathiincarboxamide der Formel (IV) 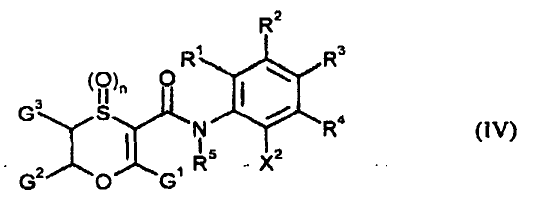 in welcher
G¹, G², G³, n, R¹, R², R³, R⁴ und R⁵ die in Anspruch 1 angegebenen Bedeutungen haben,
X² für Brom oder Iod steht,
mit Phenyl-Derivaten der Formel (VII)
X³-Z¹ (VII)
in welcher
Z¹ die in Anspruch 1 angegebenen Bedeutungen hat und
X³ für Chlor, Brom, Iod oder Trifluormethylsulfonat steht,
in Gegenwart eines Palladium- oder Nickel-Katalysators und in Gegenwart von 4,4,4',4',5,5,5',5-Octamethyl-2,2'-bis-1,3,2-dioxaborolan, gegebenenfalls in Gegenwart eines Säurebindemittels und gegebenenfalls in Gegenwart eines Verdünnungsmittels umsetzt, oder
e) Oxathiincarboxamide der Formel (Ia) 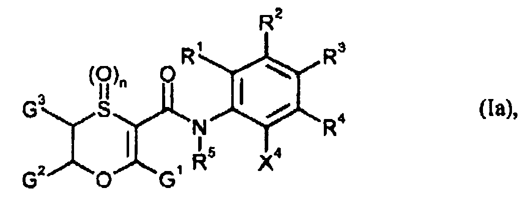 in welcher
G¹, G², G³, n, R¹, R², R³, R⁴ und R⁵ die in Anspruch 1 angegebenen Bedeutungen haben,
X⁴ für jeweils gegebenenfalls einfach oder mehrfach, gleich oder verschieden durch Halogen und/oder C₃-C₆-Cycloalkyl substituiertes C₂-C₂₀₋Alkenyl oder C₂-C₂₀-Alkinyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart eines Katalysators hydriert, oder
f) Hydroryalkyloxathiincarboxamide der Formel (VIII) 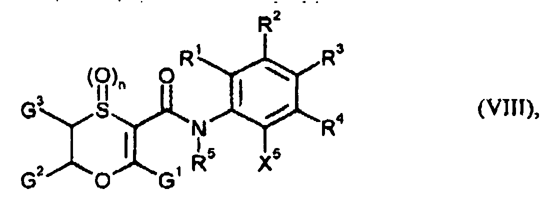 in welcher
G¹, G², G³, n, R¹, R², R³, R⁴ und R⁵ die in Anspruch 1 angegebenen Bedeutungen haben,
X⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart einer Säure dehydratisiert, oder
g) Halogenoxathiincarboxamide der Formel (IV) 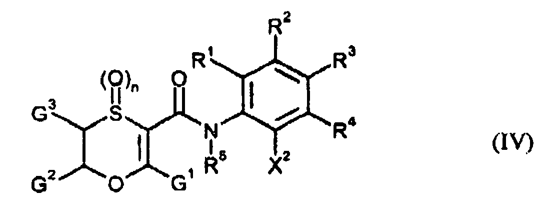 in welcher
G¹, G², G³, n, R¹, R², R³, R⁴ und R⁵ die in Anspruch 1 angegebenen Bedeutungen haben,
X² für Brom oder Iod steht,
mit einem Alkin der Formel (IX)
HC≡-A⁵ (IX),
in welcher
A⁵ für jeweils gegebenenfalls einfach oder mehrfach, gleich oder verschieden durch Halogen und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
oder einem Alken der Formel (X) 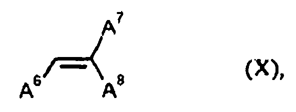 in welcher
A⁶, A⁷ und A⁸ unabhängig voneinander jeweils für Wasserstoff oder gegebenenfalls einfach oder mehrfach, gleich oder verschieden durch Halogen und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 20 nicht übersteigt,
gegebenenfalls in Gegenwart eines Verdünnungsmittels, gegebenenfalls in Gegenwart eines Säurebindemittels und in Gegenwart eines oder mehrerer Katalysatoren umsetzt, oder
h) Ketone der Formel (XI) 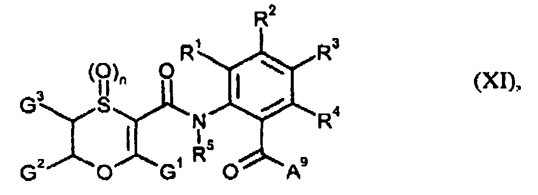 in welcher
G¹, G², G³, n, R¹, R², R³, R⁴ und R⁵ die in Anspruch 1 angegebenen Bedeutungen haben,
A⁹ für Wasserstoff oder gegebenen falls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
mit einer Phosphorverbindung der allgemeinen Formel (XII)
A¹⁰-Px (XII),
in welcher
A¹⁰ für gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen, und/oder C₁-C₄-Alkyl substituiert sein kann,
Px für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃ steht,
gegebenenfalls in Gegenwart eines Verdünnungsmittels umsetzt, oder
i) Oxathiincarboxamide der Formel (Ib) 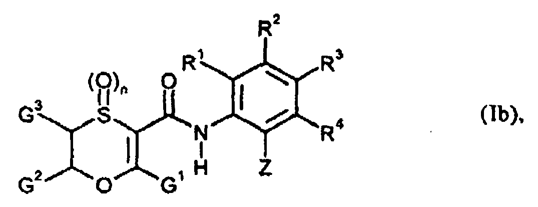 in welcher
G¹, G², G³, n, R¹, R², R³, R⁴ und Z die in Anspruch 1 angegebenen Bedeutungen haben,
mit Halogeniden der Formel (XIII)
R⁵⁻¹-X⁶ (XIII)
in welcher
R⁵⁻¹ für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)-carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁶, -CONR⁷R⁸ oder -CH₂NR⁹R¹⁰ steht,
R⁶, R⁷, R⁸, R⁹ und R¹⁰ die in Anspruch 1 angegebenen Bedeutungen haben,
X⁶ für Chlor, Brom oder Iod steht,
in Gegenwart einer Base und in Gegenwart eines Verdünnungsmittels umsetzt.

10. Mittel zur Bekämpfung unerwünschter Mikroorganismen, **gekennzeichnet durch** einen Gehalt an mindestens einem Oxathiincarboxamid der Formel (I) gemäß Anspruch 1 neben Streckmitteln und/oder oberflächenaktiven Stoffen.

11. Verwendung von Oxathiincarboxamiden der Formel (I) gemäß Anspruch 1 zur Bekämpfung unerwünschter Mikroorganismen.

12. Verfahren zur Bekämpfung unerwünschter Mikroorganismen, **dadurch gekennzeichnet, dass** man Oxathiincarboxamide der Formel (1) gemäß Anspruch 1 auf die Mikroorganismen und/oder deren Lebensraum aufbringt.

13. Verfahren zur Herstellung von Mitteln zur Bekämpfung unerwünschter Mikroorganismen, **dadurch gekennzeichnet, dass** man Oxathiincarboxamide der Formel (I) gemäß Anspruch 1 mit Streckmitteln und/oder oberflächenaktiven Stoffen vermischt.

14. Halogenoxathiincarboxamide der Formel (IV) 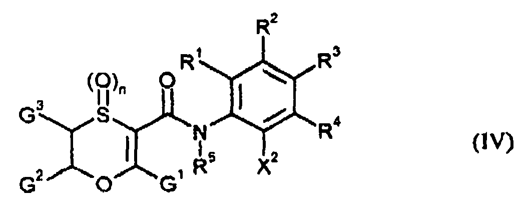 in welcher
G¹, G², G³, n, R¹, R², R³, R⁴ und R⁵ die in Anspruch 1 angegebenen Bedeutungen haben, und
X² für Brom oder Iod steht.

15. Oxathiincarboxamid-Boronsäure-Derivate der Formel (VI) 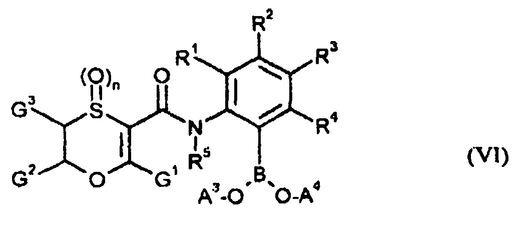 in welcher
G¹, G², G³, n, R¹, R², R³, R⁴ und R⁵ die in Anspruch 1 angegebenen Bedeutungen haben und
A³ und A⁴ jeweils für Wasserstoff oder zusammen für Tetramethylethylen stehen.

16. Hydroxyalkyloxathiincarboxamide der Formel (VIII) 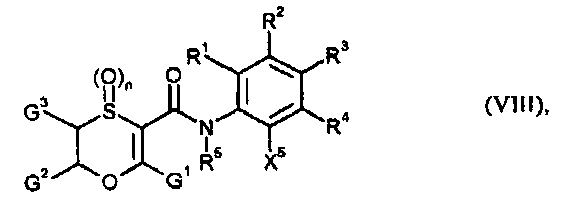 in welcher
G¹, G², G³, n, R¹, R², R³, R⁴ und R⁵ die in Anspruch 1 angegebenen Bedeutungen haben und
X⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann.

## Claims

1. Oxathiincarboxamides of the formula (I) 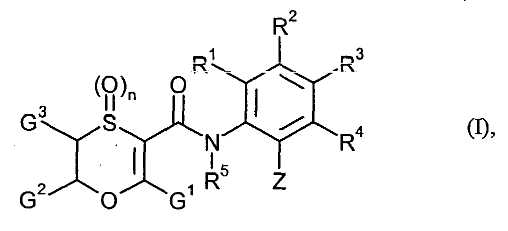 in which
G¹ represents halogen, trifluoromethyl, difluoromethyl or cyclopropyl,
G² and G³ independently of one another represent hydrogen or methyl,
n represents 0, 1 or 2,
R¹, R², R³ and R⁴ independently of one another represent hydrogen, fluorine, chlorine, methyl, isopropyl or methylthio,
R⁵ represents hydrogen, C₁-C₈-alkyl, C₁-C₆-alkylsulfinyl, C₁-C₆-alkylsulfonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulfinyl, C₁-C₄-haloalkylsulfonyl, halo-C₁-C₄-alkoxy-C₁-C₄-alkyl, C₃-C₈-halocycloalkyl having in each case 1 to 9 fluorine, chlorine and/or bromine atoms, formyl-C₁-C₃-alkyl, (C₁-C₃-alkyl)carbonyl-C₁-C₃-alkyl, (C₁-C₃-alkoxy)carbonyl-C₁-C₃-alkyl; (C₁-C₃-haloalkyl)carbonyl-C₁-C₃-alkyl, (C₁-C₃-haloalkoxy)carbonyl-C₁-C₃-alkyl having in each case 1 to 7 fluorine, chlorine and/or bromine atoms, (C₁-C₃-alkyl)carbonyl-C₁-C₃-haloalkyl, (C₁-C₃-alkoxy)carbonyl-C₁-C₃₋haloalkyl having in each case 1 to 6 fluorine, chlorine and/or bromine atoms, (C₁-C₃-haloalkyl)carbonyl-C₁-C₃-haloalkyl, (C₁-C₃-haloalkoxy)carbonyl-C₁-C₃-haloalkyl having in each case 1 to 13 fluorine, chlorine and/or bromine atoms; -COR⁶, -CONR⁷R⁸ or -CH₂NR⁹R¹⁰,
R⁶ represents hydrogen, C₁-C₈-alkyl, C₁-C₈-alkoxy, C₁-C₄-alkoxy-C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms; -COR¹¹,
R⁷ and R⁸ independently of one another represent hydrogen, C₁-C₈-alkyl, C₁-C₄-alkoxy-C₁-C₄-alkyl, C₃-C₈-cycloalkyl; C₁-C₈-haloalkyl, halo-C₁-C₄-alkoxy-C₁-C₄-alkyl, C₃-C₈-halocycloalkyl having in each case 1 to 9 fluorine, chlorine and/or bromine atoms,
R⁷ and R⁸ furthermore together with the nitrogen atom to which they are attached form a saturated heterocycle having 5 to 8 ring atoms, where the heterocycle may contain 1 or 2 further nonadjacent heteroatoms from the group consisting of oxygen, sulfur and NR¹² and is optionally mono- or poly-substituted by identical or different substituents from the group consisting of halogen and C₁-C₄-alkyl,
R⁹ and R¹⁰ independently of one another represent hydrogen, C₁-C₈-alkyl, C₃-C₈-cycloalkyl; C₁-C₈-haloalkyl, C₃-C₈-halocycloalkyl having in each case 1 to 9 fluorine, chlorine and/or bromine atoms,
R⁹ and R¹⁰ furthermore together with the nitrogen atom to which they are attached form a saturated heterocycle having 5 to 8 ring atoms, where the heterocycle may contain 1 or 2 further nonadjacent heteroatoms from the group consisting of oxygen, sulfur and NR¹² and is optionally mono- or poly-substituted by identical or different substituents from the group consisting of halogen and C₁-C₄-alkyl,
R¹¹ represents hydrogen, C₁-C₈-alkyl, C₁-C₈-alkoxy, C₁-C₄-alkoxy-C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms,
R¹² represents hydrogen or C₁-C₆-alkyl,
Z represents Z¹, Z², Z³ or Z⁴, where
Z¹ represents phenyl which is mono- to pentasubstituted by identical or different substituents,
Z² represents cycloalkyl or bicycloalkyl which is optionally mono- or poly-substituted by identical or different substituents,
Z³ represents unsubstituted C₄-C₂₀-alkyl or represents C₁-C₂₀-alkyl which is mono- or polysubstituted by identical or different substituents from the group consisting of halogen and C₃-C₆-cycloalkyl, where the cycloalkyl moiety for its part may optionally be mono- or polysubstituted by identical or different substituents from the group consisting of halogen and C₁-C₄-alkyl,
Z⁴ represents C₂-C₂₀-alkenyl or C₂-C₂₀-alkynyl which is in each case optionally mono- or polysubstituted by identical or different substituents from the group consisting of halogen and C₃-C₆-cycloalkyl, where the cycloalkyl moiety for its part may optionally be mono- or polysubstituted by identical or different substituents from the group consisting of halogen and C₁-C₄-alkyl,
or
R¹, R² and R³ independently of one another represent hydrogen or fluorine and
Z and R⁴ together with the carbon atoms to which they are attached form an optionally substituted 5- or 6-membered carbocyclic or heterocyclic ring.

2. Oxathiincarboxamides of the formula (I) according to Claim 1, in which
G¹ represents fluorine, chlorine, bromine, iodine, trifluoromethyl, difluoromethyl or cyclopropyl,
G² and G³ independently of one another represent hydrogen or methyl,
n represents 0 or 2.

3. Oxathiincarboxamides of the formula (I) according to Claim 1 in which
R⁵ represents hydrogen.

4. Oxathiincarboxamides of the formula (I) according to Claim 1 in which
R¹ represents hydrogen, fluorine, chlorine or methyl,
R² represents hydrogen, fluorine, chlorine, isopropyl or methylthio,
R³ represents hydrogen, fluorine, chlorine or methyl,
R⁴ represents hydrogen, fluorine, chlorine or methyl.

5. Oxathiincarboxamides of the formula (I) according to Claim 1 in which
Z represents Z¹,
Z¹ represents phenyl which is mono- to pentasubstituted by identical or different substituents, where the substituents are selected from the list W¹
W¹ represents halogen, cyano, nitro, amino, hydroxyl, formyl, carboxyl, carbamoyl, thiocarbamoyl;
in each case straight-chain or branched alkyl, hydroxyalkyl, oxoalkyl, alkoxy, alkoxyalkyl, alkylthioalkyl, dialkoxyalkyl, alkylthio, alkylsulfinyl or alkylsulfonyl having in each case 1 to 8 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulfinyl or haloalkylsulfonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylaminocarbonyl, dialkylaminocarbonyl, arylalkylaminocarbonyl, dialkylaminocarbonyloxy having 1 to 6 carbon atoms in the respective hydrocarbon chains, alkenylcarbonyl or alkynylcarbonyl having 2 to 6 carbon atoms in the respective hydrocarbon chains;
cycloalkyl or cycloalkyloxy having in each case 3 to 6 carbon atoms;
in each case doubly attached alkylene having 3 or 4 carbon atoms, oxyalkylene having 2 or 3 carbon atoms or dioxyalkylene having 1 or 2 carbon atoms, each of which radicals is optionally mono- to tetrasubstituted by identical or different substituents from the group consisting of fluorine, chlorine, oxo, methyl, trifluoromethyl and ethyl;
or a grouping 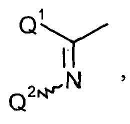 in which
Q¹ represents hydrogen, hydroxyl or alkyl having 1 to 4 carbon atoms, haloalkyl having 1 to 4 carbon atoms and 1 to 9 fluorine, chlorine and/or bromine atoms or cycloalkyl having 1 to 6 carbon atoms and
Q² represents hydroxyl, amino, methylamino, phenyl, benzyl or represents in each case optionally cyano-, hydroxyl-, alkoxy-, alkylthio-, alkylamino-, dialkylamino- or phenyl-substituted alkyl or alkoxy having 1 to 4 carbon atoms, or represents alkenyloxy or alkynyloxy having in each case 2 to 4 carbon atoms,
and also phenyl, phenoxy, phenylthio, benzoyl, benzoylethenyl, cinnamoyl, heterocyclyl or phenylalkyl, phenylalkyloxy, phenylalkylthio or heterocyclylalkyl having in each case 1 to 3 carbon atoms in the respective alkyl moieties, each of which radicals is optionally mono- to trisubstituted in the cyclic part by halogen and/or straight-chain or branched alkyl or alkoxy having 1 to 4 carbon atoms.

6. Oxathiincarboxamides of the formula (I) according to Claim 1 in which
Z represents Z²,
Z² represents cycloalkyl or bicycloalkyl having in each case 3 to 10 carbon atoms, each of which radicals is optionally mono- to tetrasubstituted by identical or different substituents from the group consisting of halogen and/or C₁-C₄-alkyl.

7. Oxathiincarboxamides of the formula (I) according to Claim 1 in which
Z represents Z³,
Z³ represents unsubstituted C₄-C₂₀-alkyl or represents C₁-C₂₀-alkyl which is monosubstituted or polysubstituted by identical or different substituents from the group consisting of fluorine, chlorine, bromine, iodine and C₃-C₆-cycloalkyl, where the cycloalkyl moiety for its part may optionally be mono- to tetrasubstituted by identical or different substituents from the group consisting of fluorine, chlorine, bromine, iodine, C₁-C₄-alkyl and/or C₁-C₄-haloalkyl.

8. Oxathiincarboxamides of the formula (I) according to Claim 1 in which
Z represents Z⁴,
Z⁴ represents C₂-C₂₀-alkenyl or C₂-C₂₀-alkynyl, each of which is mono- or polysubstituted by identical or different substituents from the group consisting of fluorine, chlorine, bromine, iodine and C₃-C₆-cycloalkyl, where the cycloalkyl moiety for its part may optionally be mono- to tetra-substituted by identical or different substituents from the group consisting of fluorine, chlorine, bromine, iodine, C₁-C₄-alkyl and C₁-C₄-haloalkyl.

9. Process for preparing the oxathiincarboxamides of the formula (1) according to Claim 1, **characterized in that**
a) oxathiincarboxylic acid derivatives of the formula (II) 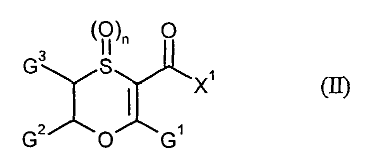 in which
G¹, G², G³ and n are as defined in Claim 1,
X¹ represents halogen or hydroxyl,
are reacted with aniline derivatives of the formula (III) 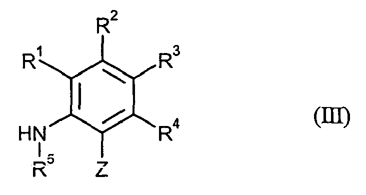 in which
R¹, R², R³, R⁴, R⁵ and Z are as defined in Claim 1,
if appropriate in the presence of a catalyst, if appropriate in the presence of a condensing agent, if appropriate in the presence of an acid binder and if appropriate in the presence of a diluent, or
b) halooxathiincarboxamides of the formula (IV) 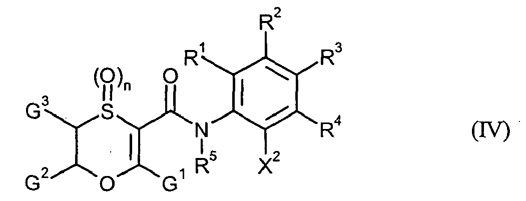 in which
G¹, G², G³, n, R¹, R², R³, R⁴ and R⁵ are as defined in Claim 1,
X² represents bromine or iodine,
are reacted with boronic acid derivatives of the formula (V) 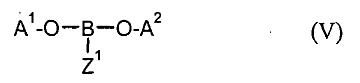 in which
Z¹ is as defined in Claim 1 and
A¹ and A² each represent hydrogen or together represent tetramethylethylene,
in the presence of a catalyst, if appropriate in the presence of an acid binder and if appropriate in the presence of a diluent, or
c) oxathiincarboxamide boronic acid derivatives of the formula (VI) 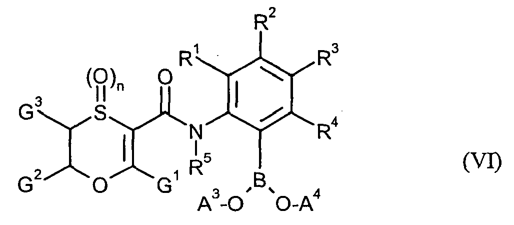 in which
G¹, G², G³, n, R¹, R², R³, R⁴ and R⁵ are as defined in Claim 1,
A³ and A⁴ each represent hydrogen or together represent tetramethylethylene,
are reacted with phenyl derivatives of the formula (VII)
X³-Z¹ (VII)
in which
Z¹ is as defined in Claim 1 and
X³ represents chlorine, bromine, iodine or trifluoromethylsulfonate,
in the presence of a catalyst, if appropriate in the presence of an acid binder and if appropriate in the presence of a diluent, or
d) halooxathiincarboxamides of the formula (IV) 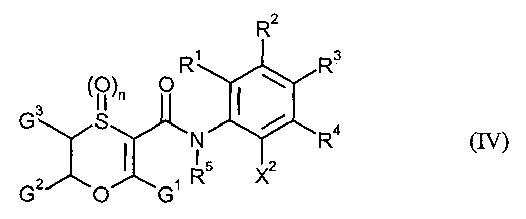 in which
G¹, G², G³, n, R¹, R², R³, R⁴ and R⁵ are as defined in Claim 1,
X² represents bromine or iodine,
are reacted with phenyl derivatives of the formula (VII)
X³-Z¹ (VII)
in which
Z¹ is as defined in Claim 1 and
X³ represents chlorine, bromine, iodine or trifluoromethylsulfonate,
in the presence of a palladium or nickel catalyst and in the presence of 4,4,4',4',5,5,5',5'-octamethyl-2,2'-bis-1,3,2-dioxaborolane, if appropriate in the presence of an acid binder and if appropriate in the presence of a diluent, or
e) oxathiincarboxamides of the formula (Ia) 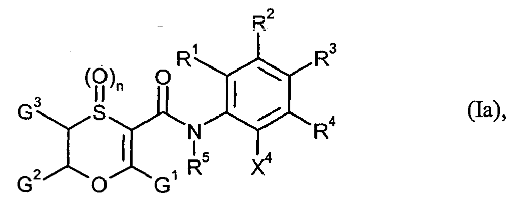 in which
G¹, G², G³, n, R¹, R², R³, R⁴ and R⁵ are as defined in Claim 1,
X⁴ represents C₂-C₂₀-alkenyl or C₂-C₂₀-alkynyl, each of which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
are hydrogenated, if appropriate in the presence of a diluent and if appropriate in the presence of a catalyst, or
f) hydroxyalkyloxathiincarboxamides of the formula (VIII) 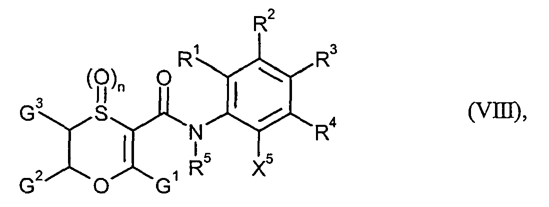 in which
G¹, G², G³, n, R¹, R², R³, R⁴ and R⁵ are as defined in Claim 1,
X⁵ represents C₂-C₂₀-hydroxyalkyl which is optionally additionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
are dehydrated, if appropriate in the presence of a diluent and if appropriate in the presence of an acid, or
g) halooxathiincarboxamides of the formula (IV) 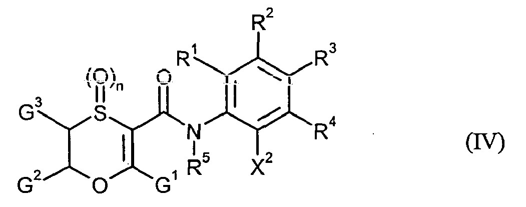 in which
G¹, G², G³, n, R¹, R², R³, R⁴ and R⁵ are as defined in Claim 1,
X² represents bromine or iodine,
are reacted with an alkyne of the formula (IX)
HC≡-A⁵ (IX),
in which
A⁵ represents C₂-C₁₈-alkyl, each of which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
or an alkene of the formula (X) 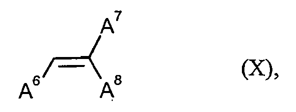 in which
A⁶, A⁷ and A⁸ independently of one another each represent hydrogen or alkyl which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl and the total number of carbon atoms of the open-chain part of the molecule does not exceed the number 20,
if appropriate in the presence of a diluent, if appropriate in the presence of an acid binder and in the presence of one or more catalysts, or
h) ketones of the formula (XI) 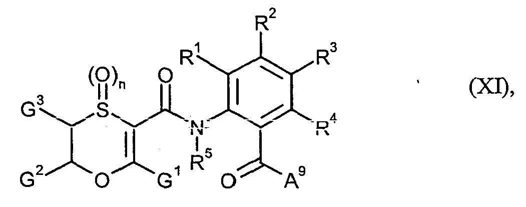 in which
G¹, G², G³, n, R¹, R², R³, R⁴ and R⁵ are as defined in Claim 1,
A⁹ represents hydrogen or C₁-C₁₈-alkyl which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
are reacted with a phosphorus compound of the formula (XII)
A¹⁰-Px (XII),
in which
A¹⁰ represents C₁-C₁₈-alkyl which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
Px represents a grouping -P⁺(C₆H₅)₃ Cl⁻, -P⁺(C₆H₅)₃ Br⁻, -P⁺(C₆H₅)₃ I⁻, -P(=O)(OCH₃)₃ or -P(=O)(OC₂H₅)₃,
if appropriate in the presence of a diluent, or
i) oxathiincarboxamides of the formula (Ib) 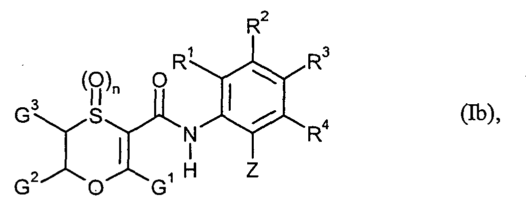 in which
G¹, G², G³, n, R¹, R², R³, R⁴ and Z are as defined in Claim 1,
are reacted with halides of the formula (XIII)
R⁵⁻¹-X⁶ (XIII)
in which
R⁵⁻¹ represents C₁-C₈-alkyl, C₁-C₆-alkylsulfinyl, C₁-C₆-alkylsulfonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulfinyl, C₁-C₄-haloalkylsulfonyl, halo-C₁-C₄-alkoxy-C₁-C₄-alkyl, C₃-C₈-halocycloalkyl having in each case 1 to 9 fluorine, chlorine and/or bromine atoms, formyl-C₁-C₃-alkyl, (C₁-C₃-alkyl)carbonyl-C₁-C₃-alkyl, (C₁-C₃-alkoxy)carbonyl-C₁-C₃-alkyl; (C₁-C₃-haloalkyl)carbonyl-C₁-C₃-alkyl, (C₁-C₃-haloalkoxy)carbonyl-C₁-C₃-alkyl having in each case 1 to 7 fluorine, chlorine and/or bromine atoms, (C₁-C₃-alkyl)carbonyl-C₁-C₃-haloalkyl, (C₁-C₃-alkoxy)carbonyl-C₁-C₃-haloalkyl having in each case 1 to 6 fluorine, chlorine and/or bromine atoms, (C₁-C₃-haloalkyl)carbonyl-C₁-C₃-haloalkyl, (C₁-C₃-haloalkoxy)carbonyl-C₁-C₃-haloalkyl having in each case 1 to 13 fluorine, chlorine and/or bromine atoms; -COR⁶, -CONR⁷R⁸ or -CH₂NR⁹R¹⁰,
R⁶, R⁷, R⁸, R⁹ and R¹⁰ are as defined in Claim 1,
X⁶ represents chlorine, bromine or iodine,
in the presence of a base and in the presence of a diluent.

10. Compositions for controlling unwanted microorganisms, **characterized in that** they comprise at least one oxathiincarboxamide of the formula (I) according to Claim 1, in addition to extenders and/or surfactants.

11. Use of oxathiincarboxamides of the formula (I) according to Claim 1 for controlling unwanted microorganisms.

12. Method for controlling unwanted microorganisms, **characterized in that** oxathiincarboxamides of the formula (I) according to Claim 1 are applied to the microorganisms and/or their habitat.

13. Process for preparing compositions for controlling unwanted microorganisms, **characterized in that** oxathiincarboxamides of the formula (I) according to Claim 1 are mixed with extenders and/or surfactants.

14. Halooxathiincarboxamides of the formula (IV) 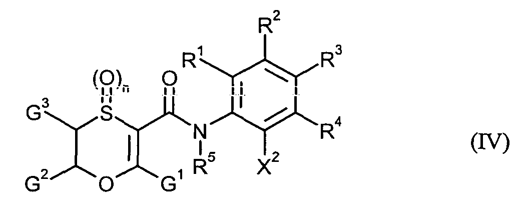 in which
G¹, G², G³, n, R¹, R², R³, R⁴ and R⁵ are as defined in Claim 1, and
X² represents bromine or iodine.

15. Oxathiincarboxamideboronic acid derivatives of the formula (VI) 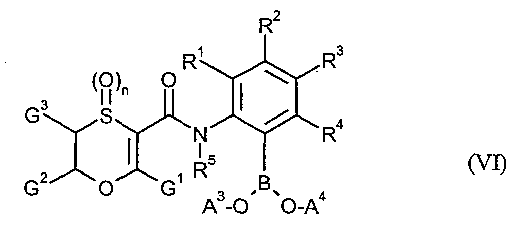 in which
G¹, G², G³, n, R¹, R², R³, R⁴ and R⁵ are as defined in Claim 1 and
A³ and A⁴ each represent hydrogen or together represent tetramethylethylene.

16. Hydroxyalkyloxathiincarboxamides of the formula (VIII) 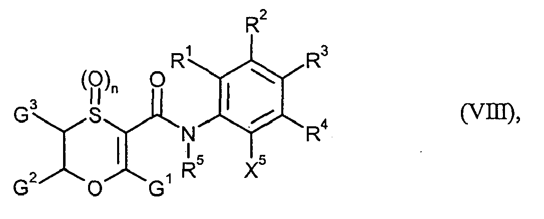 in which
G¹, G², G³, n, R¹, R², R³, R⁴ and R⁵ are as defined in Claim 1 and
X⁵ represents C₂-C₂₀-hydroxyalkyl which is optionally additionally mono- or polysubstituted by identical or different substituents from the group consisting of halogen and C₃-C₆-cycloalkyl, where the cycloalkyl moiety for its part may optionally be substituted by halogen and/or C₁-C₄-alkyl.

## Revendications

1. Oxathiine-carboxamides de formule (I) 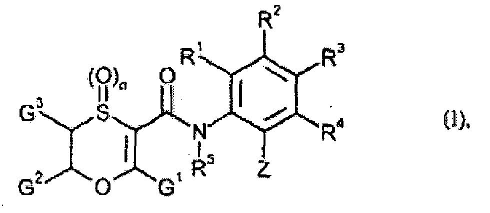 dans laquelle
G¹ représente un halogène, un reste trifluorométhyle, difluorométhyle ou cyclopropyle,
G² et G³ représentent, indépendamment l'un de l'autre, l'hydrogène ou un reste méthyle,
n a la valeur 0, 1 ou 2,
R¹, R², R³ et R⁴ représentent, indépendamment l'un de l'autre, l'hydrogène, le fluor, le chlore, un reste méthyle, isopropyle ou méthylthio,
R⁵ représente l'hydrogène, un reste alkyle en C₁ à C₈, alkylsulfinyle en C₁ à C₆, alkylsulfonyle en C₁ à C₆, (alkoxy en C₁ à C₄) - (alkyle en C₁ à C₄), cycloalkyle en C₃ à C₈, halogénalkyle en C₁ à C₆, halogénalkylthio en C₁ à C₄, halogénalkylsulfinyle en C₁ à C₄, halogénalkylsulfonyle en C₁ à C₄, halogéno(alkoxy en C₁ à C₄)-(alkyle en C₁ à C₄), halogénocycloalkyle en C₃ à C₈, ayant dans chaque cas 1 à 9 atomes de fluor, de chlore et/ou de brome, formyl-(alkyle en C₁ à C₃), (alkyle en C₁ à C₃)carbonyl-(alkyle en C₁ à C₃), (alkoxy en C₁ à C₃)carbonyl-(alkyle en C₁ à C₃) ; (halogénalkyle en C₁ à C₃)carbonyl-(alkyle en C₁ à C₃), (halogénalkoxy en C₁ à C₃)carbonyl-(alkyle en C₁ à C₃) ayant dans chaque cas 1 à 7 atomes de fluor, de chlore et/ou de brome, (alkyle en C₁ à C₃)carbonyl-(halogénalkyle en C₁ à C₃), (alkoxy en C₁ à C₃)carbonyl-(halogénalkyle en C₁ à C₃) ayant chacun 1 à 6 atomes de fluor, de chlore et/ou de brome, (halogénalkyle en C₁ à C₃)carbonyl-(halogénalkyle en C₁ à C₃), (halogénalkoxy en C₁ à C₃)carbonyl-(halogénalkyle en C₁ à C₃) ayant dans chaque cas 1 à 13 atomes de fluor, de chlore et/ou de brome ; un groupe -COR⁶, -CONR⁷R⁸ ou -CH₂NR⁹R¹⁰,
R⁶ représente l'hydrogène, un reste alkyle en C₁ à C₈, alkoxy en C₁ à C₈, (alkoxy en C₁ à C₄)-(alkyle en C₁ à C₄), cycloalkyle en C₃ à C₈, halogénalkyle en C₁ à C₆, halogénalkoxy en C₁ à C₆, halogéno-(alkoxy en C₁ à C₄)-(alkyle en C₁ à C₄), halogénocycloalkyle en C₃ à C₈, ayant dans chaque cas 1 à 9 atomes de fluor, de chlore et/ou de brome ; un groupe -COR¹¹,
R⁷ et R⁸ représentent, indépendamment l'un de l'autre, l'hydrogène, un reste alkyle en C₁ à C₈, (alkoxy en C₁ à C₄)-(alkyle en C₁ à C₄), cycloalkyle en C₃ à C₈, halogénalkyle en C₁ à C₈, halogéno-(alkoxy en C₁ à C₄)-(alkyle en C₁ à C₄), halogénocycloalkyle en C₃ à C₈, ayant dans chaque cas 1 à 9 atomes de fluor, de chlore et/ou de brome,
R⁷ et R⁸ forment en outre, conjointement avec l'atome d'azote auquel ils sont liés, un hétérocycle penta- à octogonal saturé éventuellement substitué une ou plusieurs fois identiques ou différentes par un radical halogéno ou alkyle en C₁ à C₄, l'hétérocycle pouvant contenir un ou deux autres hétéroatomes non contigus de la série oxygène, soufre ou NR¹²,
R⁹ et R¹⁰ représentent, indépendamment l'un de l'autre, l'hydrogène, un reste alkyle en C₁ à C₈, cycloalkyle en C₃ à C₈, halogénalkyle en C₁ à C₈, halogénocycloalkyle en C₃ à C₈ ayant dans chaque cas 1 à 9 atomes de fluor, de chlore et/ou de brome,
R⁹ et R¹⁰ forment en outre, conjointement avec l'atome d'azote auquel ils sont liés, un hétérocycle penta- à octogonal saturé éventuellement substitué une ou plusieurs fois identiques ou différentes par un radical halogéno ou alkyle en C₁ à C₄, l'hétérocycle pouvant contenir un ou deux autres hétéroatomes non contigus de la série oxygène, soufre ou NR¹²,
R¹¹ représente l'hydrogène, un reste alkyle en C₁ à C₈, alkoxy en C₁ à C₈, (alkoxy en C₁ à C₄)-(alkyle en C₁ à C₄), cycloalkyle en C₃ à C₈, halogénalkyle en C₁ à C₆, halogénalkoxy en C₁ à C₆, halogéno-(alkoxy en C₁ à C₄)-(alkyle en C₁ à C₄), halogénocycloalkyle en C₃ à C₈, ayant dans chaque cas 1 à 9 atomes de fluor, de chlore et/ou de brome,
R¹² représente l'hydrogène ou un reste alkyle en C₁ à C₆,
Z représente Z¹, Z², Z³ ou Z⁴, où
Z¹ représente un reste phényle substitué une à cinq fois identiques ou différentes,
Z² représente un reste cycloalkyle ou bicycloalkyle éventuellement substitué une ou plusieurs fois identiques ou différentes,
Z³ représente un reste alkyle en C₄ à C₂₀ non substitué ou un reste alkyle en C₁ à C₂₀ substitué une ou plusieurs fois identiques ou différentes par un radical halogéno et/ou cycloalkyle en C₃ à C₆, la partie cycloalkyle pouvant elle-même être éventuellement substituée une ou plusieurs fois identiques ou différentes par un radical halogéno et/ou alkyle en C₁ à C₄,
Z⁴ représente un reste alcényle en C₂ à C₂₀ ou alcynyle en C₂ à C₂₀, chacun éventuellement substitué une ou plusieurs fois identiques ou différentes par un radical halogéno et/ou cycloalkyle en C₃ à C₆, la partie cycloalkyle pouvant elle-même être éventuellement substituée une ou plusieurs fois identiques ou différentes par un radical halogéno et/ou alkyle en C₁ à C₄,
ou bien
R¹, R² et R³ représentent indépendamment les uns des autres l'hydrogène ou le fluor et
Z et R⁴ forment, conjointement avec les atomes de carbone auxquels ils sont liés, un noyau carbocyclique ou hétérocyclique penta- ou hexagonal éventuellement substitué.

2. Oxathiine-carboxamides de formule (I) suivant la revendication 1, formule dans laquelle
G¹ représente le fluor, le chlore, le brome, l'iode, un reste trifluorométhyle, difluorométhyle ou cyclopropyle,
G² et G³ représentent, indépendamment l'un de l'autre, l'hydrogène ou un reste méthyle,
n a la valeur 0 ou 2.

3. Oxathiine-carboxamides de formule (I) suivant la revendication 1, formule dans laquelle
R³ représente l'hydrogène.

4. Oxathiine-carboxamides de formule (I) suivant la revendication 1, formule dans laquelle
R¹ représente l'hydrogène, le fluor, le chlore ou un reste méthyle,
R² représente l'hydrogène, le fluor, le chlore, un reste isopropyle ou méthylthio,
R³ représente l'hydrogène, le fluor, le chlore ou un reste méthyle,
R⁴ représente l'hydrogène, le fluor, le chlore ou un reste méthyle.

5. Oxathiine-carboxamides de formule (I) suivant la revendication 1, formule dans laquelle
Z représente Z¹,
Z¹ représente un reste phényle substitué une à cinq fois identiques ou différentes, les substituants étant choisis dans la liste W¹,
W¹ représente un halogène, un groupe cyano, nitro, amino, hydroxy, formyle, carboxy, carbamoyle, thiocarbamoyle ; un reste alkyle, hydroxyalkyle, oxoalkyle, alkoxy, alkoxyalkyle, alkylthioalkyle, dialkoxyalkyle, alkylthio, alkylsulfinyle ou alkylsulfonyle, chacun linéaire ou ramifié, ayant chacun 1 à 8 atomes de carbone ;
un reste alcényle ou alcényloxy, chacun linéaire ou ramifié, et ayant chacun 2 à 6 atomes de carbone ;
un reste halogénalkyle, halogénalkoxy, halogénalkylthio, halogénalkylsulfinyle ou halogénalkylsulfonyle, chacun linéaire ou ramifié, ayant chacun 1 à 6 atomes de carbone et 1 à 13 atomes d'halogène identiques ou différents ;
un reste halogénalcényle ou halogénalcényloxy, chacun linéaire ou ramifié, ayant chacun 2 à 6 atomes de carbone et 1 à 11 atomes d'halogène identiques ou différents ;
un reste alkylamino, dialkylamino, alkylcarbonyle, alkylcarbonyloxy, alkoxycarbonyle, alkylaminocarbonyle, dialkylaminocarbonyle, arylalkylaminocarbonyle, dialkylaminocarbonyloxy, chacun linéaire ou ramifié, ayant chacun 1 à 6 atomes de carbone dans les chaînes hydrocarbonées respectives, un reste alcénylcarbonyle ou alcynylcarbonyle, ayant 2 à 6 atomes de carbone dans les chaînes hydrocarbonées respectives ;
un reste cycloalkyle ou cycloalkyloxy ayant chacun 3 à 6 atomes de carbone ;
un reste alkylène ayant 3 ou 4 atomes de carbone, oxyalkylène ayant 2 ou 3 atomes de carbone ou dioxyalkylène ayant 1 ou 2 atomes de carbone, chacun éventuellement substitué une à quatre fois identiques ou différentes par un radical fluoro, chloro, oxo, méthyle, trifluorométhyle ou éthyle ;
ou un groupement 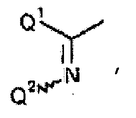 dans lequel
Q¹ représente un hydrogène, un groupe hydroxy ou un reste alkyle ayant 1 à 4 atomes de carbone, halogénalkyle ayant 1 à 4 atomes de carbone et 1 à 9 atomes de fluor, de chlore et/ou de brome ou un reste cycloalkyle ayant 1 à 6 atomes de carbone, et
Q² représente un groupe hydroxy, amino, un reste méthylamino, phényle, benzyle ou un reste alkyle ou alkoxy ayant 1 à 4 atomes de carbone, chacun éventuellement substitué par un radical cyano, hydroxy, alkoxy, alkylthio, alkylamino, dialkylamino
ou phényle, ou un reste alcényloxy ou alcynyloxy ayant chacun 2 à 4 atomes de carbone,
ainsi qu'un reste phényle, phénoxy, phénylthio, benzoyle, benzoyléthényle, cinnamoyle, hétérocyclyle ou phénylalkyle, chacun éventuellement substitué une à trois fois dans la partie cyclique par un radical halogéno et/ou alkyle ou alkoxy linéaire ou ramifié ayant 1 à 4 atomes de carbone, un reste phénylalkyloxy, phénylalkylthio ou hétérocyclylalkyle ayant chacun 1 à 3 atomes de carbone dans les parties alkyle respectives.

6. Oxathiine-carboxamides de formule (I) suivant la revendication 1, formule dans laquelle
Z représente Z²,
Z² représente un reste cycloalkyle ou bicycloalkyle, chacun éventuellement substitué une à quatre fois identiques ou différentes par un radical halogéno et/ou alkyle en C₁ à C₄ et ayant chacun 3 à 10 atomes de carbone.

7. Oxathiine-carboxamides de formule (I) suivant la revendication 1, formule dans laquelle
Z représente Z³,
Z³ représente un reste alkyle en C₄ à C₂₀ non substitué ou un reste alkyle en C₁ à C₂₀ substitué une ou plusieurs fois identiques ou différentes par un radical fluoro, chloro, bromo, iodo et/ou cycloalkyle en C₃ à C₆, la partie cycloalkyle pouvant elle-même éventuellement être substituée une à quatre fois identiques ou différentes par un radical fluoro, chloro, bromo, iodo, alkyle en C₁ à C₄ et/ou halogénalkyle en C₁ à C₄.

8. Oxathiine-carboxamides de formule (I) suivant la revendication 1, formule dans laquelle
Z représente Z⁴,
Z⁴ représente un reste alcényle en C₂ à C₂₀ ou alcynyle en C₂ à C₂₀, chacun éventuellement substitué une ou plusieurs fois identiques ou différentes par un radical fluoro, chloro, bromo, iodo et/ou cycloalkyle en C₃ à C₆, la partie cycloalkyle pouvant elle-même être éventuellement substituée une à quatre fois identiques ou différentes par un radical fluoro, chloro, bromo, iodo, alkyle en C₁ à C₄ et/ou halogénalkyle en C₁ à C₄.

9. Procédé de production des oxathiine-carboxamides de formule (I) suivant la revendication 1, **caractérisé en ce que**
a) on fait réagir des dérivés d'acides oxathiine-carboxyliques de formule (II) 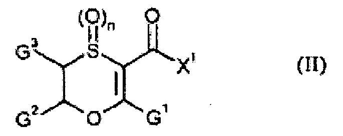 dans laquelle
G¹, G², G³ et n ont les définitions indiquées dans la revendication 1,
X¹ représente un halogène ou un groupe hydroxy,
avec des dérivés d'aniline de formule (III) 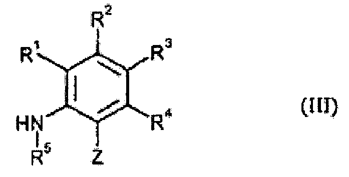 dans laquelle
R¹, R², R³, R⁴, R⁵ et Z ont les définitions indiquées dans la revendication 1,
éventuellement en présence d'un catalyseur, le cas échéant en présence d'un agent de condensation, éventuellement en présence d'un accepteur d'acide et, le cas échéant, en présence d'un diluant, ou bien
b) on fait réagir des halogénoxathiine-carboxamides de formule (IV) 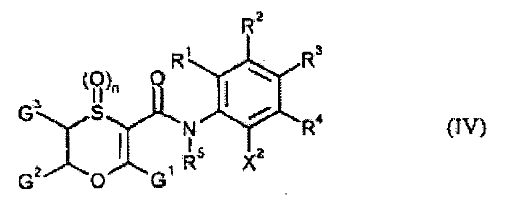 dans laquelle
G¹, G², G³, n, R¹, R², R³, R⁴, et R⁵ ont les définitions indiquées dans la revendication 1,
X² représente le brome ou l'iode,
avec des dérivés d'acide boronique de formule (V) 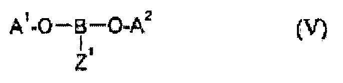 dans laquelle
Z¹ a les définitions indiquées dans la revendication 1, et
A¹ et A² représentent chacun l'hydrogène ou forment ensemble un groupe tétraméthyléthylène,
en présence d'un catalyseur, éventuellement en présence d'un accepteur d'acide et, le cas échéant, en présence d'un diluant, ou bien
c) on fait réagir des dérivés d'acides oxyathiine-carboxamide-boroniques de formule (VI) 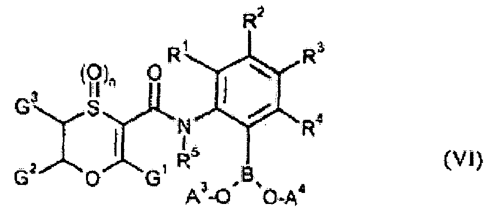 dans laquelle
G¹, G², G³, n, R¹, R², R³, R⁴, et R⁵ ont les définitions indiquées dans la revendication 1,
A³ et A⁴ représentent chacun l'hydrogène ou forment ensemble un groupe tétraméthyléthylène,
avec des dérivés phényliques de formule (VII)
X³-Z¹ (VII)
dans laquelle
Z¹ a les définitions indiquées dans la revendication 1, et
X³ représente le chlore, le brome, l'iode ou un groupe trifluorométhylsulfonate,
en présence d'un catalyseur, éventuellement en présence d'un accepteur d'acide et, le cas échéant, en présence d'un diluant, ou bien
d) on fait réagir des halogénoxathiine-carboxamides de formule (IV) 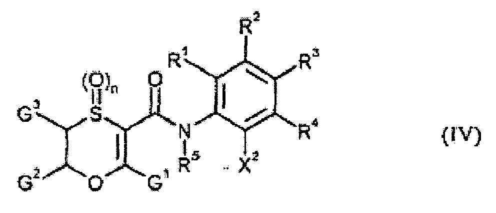 dans laquelle
G¹, G², G³, n, R¹, R², R³, R⁴, et R⁵ ont les définitions indiquées dans la revendication 1,
X² représente le brome ou l'iode,
avec des dérivés phényliques de formule (VII)
X³-Z¹ (VII)
dans laquelle
Z¹ a les définitions indiquées dans la revendication 1, et
X³ représente le chlore, le brome, l'iode ou un groupe trifluorométhylsulfonate,
en présence d'un catalyseur au palladium ou au nickel ou en présence de 4,4,4',4',5,5,5',5'-octaméthyl-2,2'-bis-1,3,2-dioxaborolane, éventuellement en présence d'un accepteur d'acide et, le cas échéant, en présence d'un diluant, ou bien
e) on fait hydrogéner des oxathiine-carboxamides de formule (Ia) 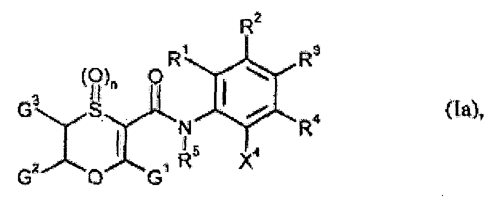 dans laquelle
G¹, G², G³, n, R¹, R², R³, R⁴, et R⁵ ont les définitions indiquées dans la revendication 1,
X⁴ représente un reste alcényle en C₂ à C₂₀ ou alcynyle en C₂ à C₂₀, chacun éventuellement substitué une ou plusieurs fois identiques ou différentes par un radical halogéno et/ou cycloalkyle en C₃ à C₆, la partie cycloalkyle pouvant elle-même éventuellement être substituée par un radical halogéno et/ou alkyle en C₁ à C₄,
éventuellement en présence d'un diluant et, le cas échéant, en présence d'un catalyseur, ou bien
f) on déshydrate des hydroxyalkyloxathiine-carboxamides de formule (VIII) 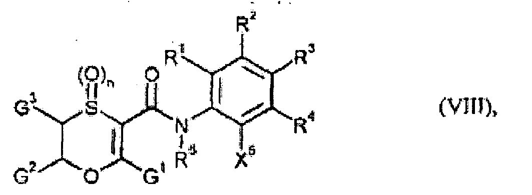 dans laquelle
G¹, G², G³, n, R¹, R², R³, R⁴, et R⁵ ont les définitions indiquées dans la revendication 1,
X⁵ représente un reste hydroxyalkyle en C₂ à C₂₀ éventuellement substitué en outre une ou plusieurs fois identiques ou différentes par un radical halogéno et/ou cycloalkyle en C₃ à C₆, la partie cycloalkyle pouvant elle-même éventuellement être substituée par un radical halogéno et/ou alkyle en C₁ à C₄,
éventuellement en présence d'un diluant et, le cas échéant, en présence d'un acide, ou bien
g) on fait réagir des halogénoxathiine-carboxamides de formule (IV) 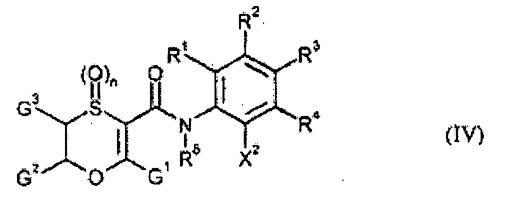 dans laquelle
G¹, G², G³, n, R¹, R², R³, R⁴, et R⁵ ont les définitions indiquées dans la revendication 1,
X² représente le brome ou l'iode,
avec un alcyne de formule (IX)
HC≡-A⁵ (IX),
dans laquelle
A⁵ représente un reste alkyle en C₂ à C₁₈ éventuellement substitué une ou plusieurs fois identiques ou différentes par un radical halogéno et/ou cycloalkyle en C₃ à C₆, la partie cycloalkyle pouvant elle-même être éventuellement substituée par un radical halogéno et/ou alkyle en C₁ à C₄,
ou avec un alcène de formule (X) 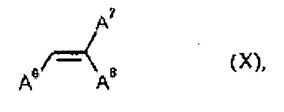 dans laquelle
A⁶, A⁷ et A⁸ représentent indépendamment les uns des autres, dans chaque cas, l'hydrogène ou un reste alkyle éventuellement substitué une ou plusieurs fois identiques ou différentes par un radical halogéno et/ou cycloalkyle en C₃ à C₆, la partie cycloalkyle pouvant elle-même être éventuellement substituée par un radical halogéno et/ou un reste alkyle en C₁ à C₄ et le nombre total d'atomes de carbone de la partie de la molécule en chaîne ouverte ne dépassant pas 20,
le cas échéant en présence d'un diluant, éventuellement en présence d'un accepteur d'acide ou en présence d'un ou plusieurs catalyseurs, ou bien
h) on fait réagir des cétones de formule (XI) 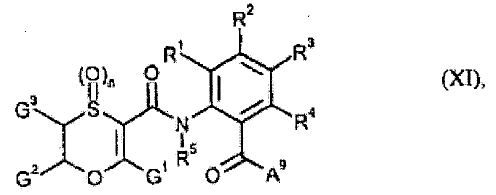 dans laquelle
G¹, G², G³, n, R¹, R², R³, R⁴, et R⁵ ont les définitions indiquées dans la revendication 1,
A⁹ représente l'hydrogène ou un reste alkyle en C₁ à C₁₈, éventuellement substitué une ou plusieurs fois identiques ou différentes par un radical halogéno et/ou cycloalkyle en C₃ à C₆, la partie cycloalkyle pouvant elle-même être éventuellement substituée par un radical halogéno et/ou alkyle en C₁ à C₄,
avec un composé de phosphore de formule générale (XII)
A¹⁰-Px (XII),
dans laquelle
A¹⁰ représente un reste alkyle en C₁ à C₁₈, éventuellement substitué une ou plusieurs fois identiques ou différentes par un radical halogéno et/ou cycloalkyle en C₃ à C₆, la partie cycloalkyle pouvant elle-même être éventuellement substituée par un radical halogéno et/ou alkyle en C₁ à C₄,
Px représente un groupement -P⁺(C₆H₅)₃Cl⁻, -P⁺(C₆H₅)₃Br⁻, -P⁺(C₆H₅)₃I⁻, -P (=O) (OCH₃)₃ ou -P(=O) (OC₂H₅)₃,
éventuellement en présence d'un diluant, ou bien
i) on fait réagir des oxathiine-carboxamides de formule (Ib) 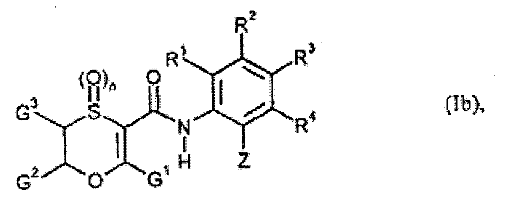 dans laquelle
G¹, G², G³, n, R¹, R², R³, R⁴, et Z ont les définitions indiquées dans la revendication 1,
avec des halogénures de formule (XIII)
R⁵⁻¹-X⁶ (XIII)
dans laquelle
R⁵⁻¹ représente un reste alkyle en C₁ à C₈, alkylsulfinyle en C₁ à C₆, alkylsulfonyle en C₁ à C₆, (alkoxy en C₁ à C₄)-(alkyle en C₁ à C₄), cycloalkyle en C₃ à C₈ ; halogénalkyle en C₁ à C₆, halogénalkylthio en C₁ à C₄, halogénalkylsulfinyle en C₁ à C₄, halogénalkylsulfonyle en C₁ à C₄, halogéno-(alkoxy en C₁ à C₄)-(alkyle en C₁ à C₄), halogénocycloalkyle en C₃ à C₈, ayant chacun 1 à 9 atomes de fluor, de chlore et/ou de brome, formyl-(alkyle en C₁ à C₃), (alkyle en C₁ à C₃)carbonyl-(alkyle en C₁ à C₃), (alkoxy en C₁ à C₃)carbonyl-(alkyle en C₁ à C₃) ; (halogénalkyle en C₁ à C₃)carbonyl-(alkyle en C₁ à C₃), (halogénalkoxy en C₁ à C₃)carbonyl-(alkyle en C₁ à C₃) ayant dans chaque cas 1 à 7 atomes de fluor, de chlore et/ou de brome, (alkyle en C₁ à C₃)carbonyl-(halogénalkyle en C₁ à C₃), (alkoxy en C₁ à C₃)carbonyl-(halogénalkyle en C₁ à C₃) ayant dans chaque cas 1 à 6 atomes de fluor, de chlore et/ou de brome, (halogénalkyle en C₁ à C₃)carbonyl-(halogénalkyle en C₁ à C₃), (halogénalkoxy en C₁ à C₃)carbonyl-(halogénalkyle en C₁ à C₃) ayant dans chaque cas 1 à 13 atomes de fluor, de chlore et/ou de brome ; un groupe -COR⁶, -CONR⁷R⁸ ou -CH₂NR⁹R¹⁰,
R⁶, R⁷, R⁸, R⁹ et R¹⁰ ont les définitions indiquées dans la revendication 1,
X⁶ représente le chlore, le brome ou l'iode,
en présence d'une base et, le cas échéant, en présence d'un diluant.

10. Composition destinée à combattre des micro-organismes indésirables, **caractérisée par** une teneur en au moins un oxathiine-carboxamide de formule (I) suivant la revendication 1, à côté de diluants et/ou d'agents tensio-actifs.

11. Utilisation d'oxathiine-carboxamides de formule (I) suivant la revendication 1 pour combattre des micro-organismes indésirables.

12. Procédé pour combattre des micro-organismes indésirables, **caractérisé en ce qu'**on épand des oxathiine-carboxamides de formule (I) suivant la revendication 1 sur les micro-organismes et/ou sur leur milieu.

13. Procédé de préparation de compositions destinées à combattre des micro-organismes indésirables, **caractérisé en ce qu'**on mélange des oxathiine-carboxamides de formule (I) suivant la revendication 1 avec des diluants et/ou des agents tensio-actifs.

14. Halogénoxathiine-carboxamides de formule (IV) 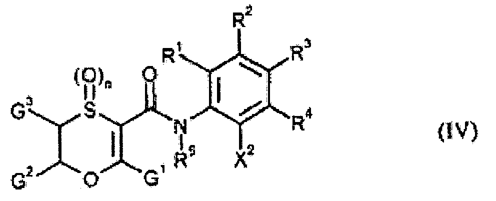 dans laquelle
G¹, G², G³, n, R¹, R², R³, R⁴, et R⁵ ont les définitions indiquées dans la revendication 1, et
X² représente le brome ou l'iode.

15. Dérivés d'acides oxathiine-carboxamide-boroniques de formule (VI) 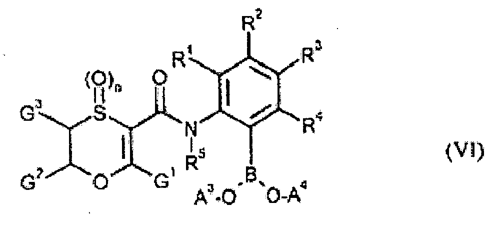 dans laquelle
G¹, G², G³, n, R¹, R², R³, R⁴, et R⁵ ont les définitions indiquées dans la revendication 1, et
A³ et A⁴ représentent chacun l'hydrogène ou forment conjointement un groupe tétraméthyléthylène.

16. Hydroxyalkyloxathiine-carboxamides de formule (VIII) 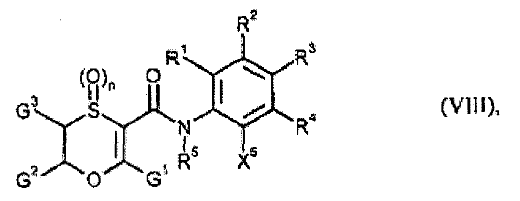 dans laquelle
G¹, G², G³, n, R¹, R², R³, R⁴, et R⁵ ont les définitions indiquées dans la revendication 1, et
X⁵ représente un reste hydroxyalkyle en C₂ à C₂₀, éventuellement substitué en outre une ou plusieurs fois identiques ou différentes par un radical halogéno et/ou cycloalkyle en C₃ à C₆, la partie cycloalkyle pouvant elle-même éventuellement être substituée par un radical halogéno et/ou alkyle en C₁ à C₄.
